# EUROPEAN PATENT SPECIFICATION

(11) **EP 4 380 928 B1**
(45) Date of publication and mention of the grant of the patent: **01.10.2025**
(21) Application number: 22761764.4
(22) Date of filing: 04.08.2022
(51) Int. Cl.: C07D 401/04, C07D 401/14, C07D 417/04, A61K 31/4709, A61K 31/506, A61P 35/00

(54) **PPARG INVERSE AGONISTS AND USES THEREOF**
PPARG-INVERSE AGONISTEN UND VERWENDUNGEN DAVON
AGONISTES INVERSES DE PPARG ET LEURS UTILISATIONS

(30) Priority: 05.08.2021 US 202163229861 P
(43) Date of publication of application: 12.06.2024
(73) Proprietor: Flare Therapeutics Inc., Cambridge, MA 02142 (US)
(72) Inventor: WILSON, Jonathan E., Arlington, MA 02474 (US); AUDIA, James E., Chicago, IL 60618 (US); STUCKEY, Jacob I., Framingham, MA 01701 (US); SCOTT, Mark, Cambridge, MA 02142 (US)
(74) Representative: Potter Clarkson
(86) International application number: PCT/US2022/039385
(87) International publication number: WO 2023/014861

(56) References cited:
- WO-A1-2011/054433
- WO-A1-2017/061957
- WO-A1-2022/187203
- CN-B- 107 522 657

## Description

### RELATED APPLICATIONS

This application claims the benefit of priority to U.S. Provisional Application No. 63/229,861, filed August 5, 2021.

### BACKGROUND

PPARgamma (PPARG) is a type II ligand-dependent nuclear hormone receptor (belonging to the PPAR nuclear receptor subfamily) that functions as an obligate heterodimer with retinoid X receptors (RXRs). PPARG is predominantly expressed in adipose tissue, colon, macrophages and the luminal layers of the urothelium. PPARG is known as a master regulator of adipogenesis, functioning to regulate adipocyte differentiation, fatty acid storage and glucose metabolism. PPARG has also been shown to play an important role in the metabolism and inflammation of macrophages, where it is induced by IL4 and controls glutamine metabolism. In the normal urothelium, PPARG is critical for its homeostasis and regeneration.

The role for PPARG in cancer was originally inferred from genomic studies that identified a PAX8-PPARG chromosomal rearrangement in follicular thyroid carcinomas. More recently, PPARG has been found to be over-expressed and genetically altered in the luminal subtype of urothelial cancer. This is consistent with reports that long-term use of PPARG agonists is associated with an increased incidence of urothelial cancer. Most urothelial cancers are urothelial carcinoma, which are classified as either non-muscle-invasive urothelial cancer (NMIUC, 70%), muscle-invasive urothelial cancer (MIUC, 25%) or metastatic urothelial cancer (MUC, 5%). MIUC is usually diagnosed de novo but may arise from the 10 to 20% of NMIUC cases that eventually progress. MIUC is a heterogeneous and aggressive disease, associated with a five-year survival rate of 60% for patients with localized disease and less than 10% for patients with distant metastases. Molecular understanding of NMIUC and MIUC has improved significantly, including the association between molecular subtypes and urothelial differentiation. Several molecular classes of MIUC have been proposed, whereby an activated PPARG signature features prominently in the luminal subtypes. First-line treatment is chemotherapy with several options in chemo-ineligible or second line, but treatment options are limited with poor overall survival rates. WO 2022/187203 relates to compounds, and pharmaceutical acceptable salts thereof, which may be useful in treating a variety of conditions associated with PPARG. CN 107522657 relates to compounds with multiple PPAR agonistic activity and preparation methods and application thereof, and to screening of a new drug for the prevention and/or treatment of metabolic disorders. WO 2017/061957 relates to quinolines and 5,6,7,8-tetrahydroacridines or pharmaceutically acceptable forms or prodrugs thereof, that are inhibitors of methyl transferases such as protein lysine methyltransferases and more particularly SMYD3. WO 2011/054433 relates to hetarylaminoquinoline derivatives which are inhibitors of ATP consuming proteins.

The need exists to develop effective PPARG modulators for treating cancers such as NMIUC, MIUC, and MUC, and related conditions.

Provided herein are compounds having the Formula **I:** 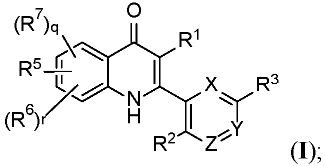 and pharmaceutically acceptable salts and compositions thereof, wherein X, Y, Z, R¹, R², R³, R⁴, R⁵, R⁶, R⁷, q and r are as described herein. In one aspect, the disclosed compounds of Formula **I** and pharmaceutically acceptable salts thereof modulate PPARG (e.g., as agonists such as inverse agonists), and are useful in a variety of therapeutic applications such as, for example, in treating cancer. As such, their uses for treating diseases responsive to the inhibition of PPARG are included. Any references to (methods of) treatment in the subsequent paragraphs of this description are to be interpreted as references to the compounds, pharmaceutical compositions and medicaments of the present invention for use in a method for treatment of the human (or animal) body by therapy (or for diagnosis).

Pharmaceutical compositions comprising the compounds and pharmaceutically acceptable salts of the disclosed compounds of Formula **I,** as well as methods for their preparation are also included.

### DETAILED DESCRIPTION

### 1. General Description of Compounds

In a first embodiment, provided herein is a compound of Formula **I:** 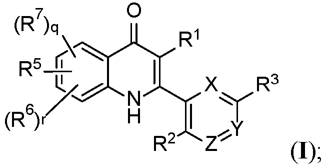 or a pharmaceutically acceptable salt thereof, wherein:
X, Y, and Z are each independently N or -CR⁴, wherein at least one of X, Y, or Z is N;
R¹ is hydrogen, halo, (C₁-C₄)alkyl, or hydroxyl;
R² is halo, -SR^{g}, -SOR^{g}, -SO₂R^{g}, or -OR^{g};
R³ is cyano or nitro;
R⁴ is hydrogen, halo, (C₁-C₄)alkyl, (C₁-C₄)alkoxy, or hydroxyl;
R⁵ is halo, halo(C₁-C₄)alkyl, or cyano;
R⁶ is halo, halo(C₁-C₄)alkyl, (C₁-C₄)alkyl, or cyano;
R⁷ is halo, (C₁-C₄)alkyl, (C₁-C₄)alkoxy, halo(C₁-C₄)alkyl, halo(C₁-C₄)alkoxy, -(C₁-C₄)alkylOR^{a}, -(C₁-C₄)alkylC(O)R^{a}, -(C₁-C₄)alkylC(O)OR ^{a}, -C(O)NR^{a}R^{b}, -(C₁-C₄)alkylC(O)NR^{a}R^{b}, -C(O)R^{a}, -C(O)OR^{a}, -NR^{a}R^{b}, -(C₁-C₄)alkylNR^{a}R^{b}, -C(O)NR^{a}SO₃H, - NR^{a}C(O)R^{b}, -NR^{a}C(O)OR^{b}, -NR^{a}C(S)OR^{b}, -NR^{c}C(O)N^{a}R^{b}, -NR^{c}C(S)NR^{a}R^{b}, - NR^{c}S(O)₂NR^{a}R^{b}, -C(S)R^{a}, -S(O)₂R^{a}, -S(O)R^{a}, -C(S)OR^{a}, -C(S)NR^{a}R^{b}, -NR^{a}C(S)R^{b}, -SR^{a}, phenyl, 4- to 6-membered heterocyclyl, and 5- to 7-membered heteroaryl, wherein each of said phenyl, 4- to 6-membered heterocyclyl, and 5- to 7-membered heteroaryl are optionally and independently substituted with 1 to 3 groups selected from R⁸;
R⁸ is selected from halo, (C₁-C₄)alkyl, halo(C₁-C₄)alkyl, (C₁-C₄)alkoxy, halo(C₁-C₄)alkoxy, nitro, oxo, cyano, -(C₁-C₄)alkylOR^{d}, -(C₁-C₄)alkylC(O)R^{d}, -(C₁-C4)alkylC(O)OR^{d}, -C(O)NR^{d}R^{e}, -(C1-C4)alkylC(O)NR^{d}R^{e}, -C(O)R^{d}, -C(O)OR^{d}, -NR^{d}R^{e}, - (C₁-C₄)alkylNR^{d}R^{e}, -C(O)NR^{d}SO₃H, -NR^{d}C(O)R^{e}, -NR^{d}C(O)OR^{e}, -NR^{d}C(S)OR^{e}, - NR^{f}C(O)N^{d}R^{e}, -NR^{f}C(S)NR^{d}R^{e}, -NR^{f}S(O)₂NR^{d}R^{e}, -C(S)R^{d}, -S(O)₂R^{d}, -S(O)R^{d}, -C(S)OR^{d}, - C(S)NR^{d}R^{e}, -NR^{d}C(S)R^{e}, and -SR^{d};
R^{a}, R^{b}, R^{c}, R^{d}, R^{e}, R^{f}, and R^{g} are each independently hydrogen or (C₁-C₄)alkyl optionally substituted with 1 or 2 -NR'R" groups wherein R' and R'' are each independently selected from hydrogen, (C₁-C₄)alkyl, and halo(C₁-C₄)alkyl; and
q and r are each independently 0 or 1.

### 2. Definitions

When used in connection to describe a chemical group that may have multiple points of attachment, a hyphen (-) designates the point of attachment of that group to the variable to which it is defined. For example, -NR^{b}C(O)OR^{c} and -NR^{b}C(S)OR^{c} mean that the point of attachment for this group occurs on the nitrogen atom.

The terms "halo" and "halogen" refer to an atom selected from fluorine (fluoro, -F), chlorine (chloro, -Cl), bromine (bromo, -Br), and iodine (iodo, -I).

The term "alkyl" when used alone or as part of a larger moiety, such as "haloalkyl", and the like, means saturated straight-chain or branched monovalent hydrocarbon radical.

"Alkoxy" means an alkyl radical attached through an oxygen linking atom, represented by -O-alkyl. For example, "(C₁-C₄)alkoxy" includes methoxy, ethoxy, proproxy, and butoxy.

The term "haloalkyl" includes mono, poly, and perhaloalkyl groups where the halogens are independently selected from fluorine, chlorine, bromine, and iodine.

"Haloalkoxy" is a haloalkyl group which is attached to another moiety via an oxygen atom such as, e.g., -OCHF₂ or -OCF₃.

The term oxo means the group =O.

The term "5- to 7-membered heteroaryl" used alone or as part of a larger moiety refers to a 5- to 7-membered aromatic radical containing 1-4 heteroatoms, which may be independently selected from N, O, and S. Monocyclic heteroaryl includes, for example, thienyl, furanyl, pyrrolyl, imidazolyl, pyrazolyl, triazolyl, tetrazolyl, oxazolyl, isoxazolyl, triazinyl, tetrazinyl, oxadiazolyl, thiazolyl, isothiazolyl, thiadiazolyl, pyridyl, pyridazinyl, pyrimidinyl, pyrazinyl, etc. Optional substituents on a heteroaryl group may be present on any substitutable position and, include, *e.g.*, the position at which the heteroaryl is attached.

The term "4- to 6-membered heterocyclyl" means a 4- to 6-membered saturated or partially unsaturated heterocyclic ring containing 1 to 4 heteroatoms, which may be independently selected from N, O, and S. A heterocyclyl ring can be attached to its pendant group at any heteroatom or carbon atom that results in a stable structure. Examples of monocyclic saturated or partially unsaturated heterocyclic radicals include, without limitation, tetrahydrofuranyl, tetrahydrothienyl, tetrahydropyranyl, pyrrolidinyl, pyrrolidonyl, piperidinyl, oxazolidinyl, piperazinyl, dioxanyl, oxetanyl, dioxolanyl, morpholinyl, thiomorpholinyl, dihydrofuranyl, dihydropyranyl, dihydropyridinyl, tetrahydropyridinyl, dihydropyrimidinyl, and tetrahydropyrimidinyl. Optional substituents on a heterocyclyl group may be present on any substitutable position and, include, *e.g.*, the position at which the heterocyclyl is attached.

Compounds having one or more chiral centers can exist in various stereoisomeric forms. Stereoisomers are compounds that differ only in their spatial arrangement. Stereoisomers include all diastereomeric, enantiomeric, and epimeric forms as well as racemates and mixtures thereof. When the stereochemical configuration at a chiral center in a compound having one or more chiral centers is depicted by its chemical name (e.g., where the configuration is indicated in the chemical name by "R" or "S") or structure (e.g., the configuration is indicated by "wedge" bonds), the enrichment of the indicated configuration relative to the opposite configuration is greater than 50%, 60%, 70%, 80%, 90%, 99% or 99.9%. "Enrichment of the indicated configuration relative to the opposite configuration" is a mole percent and is determined by dividing the number of compounds with the indicated stereochemical configuration at the chiral center(s) by the total number of all of the compounds with the same or opposite stereochemical configuration in a mixture.

When a disclosed compound is named or depicted by structure without indicating stereochemistry, it is understood that the name or the structure encompasses one of the possible stereoisomers or geometric isomers free of the others, or a mixture of the encompassed stereoisomers or geometric isomers.

The disclosed compounds may exist in one or more tautomeric forms, such as those below, and are included herein.

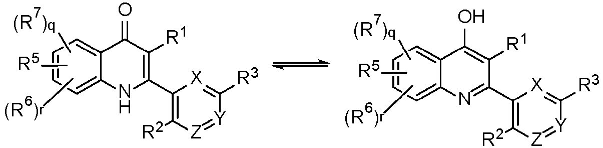

The terms "subject" and "patient" may be used interchangeably, and means a mammal in need of treatment, *e.g.*, companion animals (*e.g.*, dogs, cats, and the like), farm animals (*e.g.*, cows, pigs, horses, sheep, goats and the like) and laboratory animals (*e.g.*, rats, mice, guinea pigs and the like). Typically, the subject is a human in need of treatment.

The term "inhibit," "inhibition" or "inhibiting" includes a decrease in the baseline activity of a biological activity or process.

As used herein, the terms "treatment," "treat," and "treating" refer to reversing, alleviating, delaying the onset of, or inhibiting the progress of a disease or disorder, or one or more symptoms thereof, as described herein. In some aspects, treatment may be administered after one or more symptoms have developed, *i.e.*, therapeutic treatment. In other aspects, treatment may be administered in the absence of symptoms. For example, treatment may be administered to a susceptible individual prior to the onset of symptoms (*e.g*., in light of a history of symptoms and/or in light of exposure to a particular organism, or other susceptibility factors), *i.e.*, prophylactic treatment. Treatment may also be continued after symptoms have resolved, for example to delay their recurrence.

The term "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions described herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

For use in medicines, the salts of the compounds described herein refer to non-toxic "pharmaceutically acceptable salts." Pharmaceutically acceptable salt forms include pharmaceutically acceptable acidic/anionic or basic/cationic salts. Suitable pharmaceutically acceptable acid addition salts of the compounds described herein include e.g., salts of inorganic acids (such as hydrochloric acid, hydrobromic, phosphoric, nitric, and sulfuric acids) and of organic acids (such as, acetic acid, benzenesulfonic, benzoic, methanesulfonic, and p-toluenesulfonic acids). Compounds of the present teachings with acidic groups such as carboxylic acids can form pharmaceutically acceptable salts with pharmaceutically acceptable base(s). Suitable pharmaceutically acceptable basic salts include e.g., ammonium salts, alkali metal salts (such as sodium and potassium salts) and alkaline earth metal salts (such as magnesium and calcium salts). Compounds with a quaternary ammonium group also contain a counteranion such as chloride, bromide, iodide, acetate, perchlorate and the like. Other examples of such salts include hydrochlorides, hydrobromides, sulfates, methanesulfonates, nitrates, benzoates and salts with amino acids such as glutamic acid.

The term "effective amount" or "therapeutically effective amount" refers to an amount of a compound described herein that will elicit a desired or beneficial biological or medical response of a subject e.g., a dosage of between 0.01 - 100 mg/kg body weight/day.

### 3. Compounds

In a second embodiment, the compound of Formula **I** is of the Formula **II:** 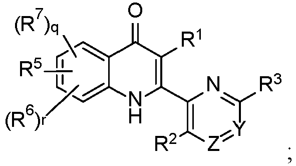 or a pharmaceutically acceptable salt thereof, wherein the variables are as described above for Formula **I.**

In a third embodiment, the compound of Formula **I** is of the Formula **III:** 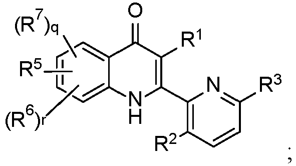 or a pharmaceutically acceptable salt thereof, wherein the variables are as described above for Formula **I.**

In a fourth embodiment, R² in the compound of Formula **I, II,** or **III,** or a pharmaceutically acceptable salt thereof is halo, -S(C₁-C₄)alkyl, -SO(C₁-C₄)alkyl, -SO₂(C₁-C₄)alkyl, or -O(C₁-C₄)alkylN[(C₁-C₄)alkyl]₂, wherein the remaining variables are as described above for Formula **I.** Alternatively, as part of a fourth embodiment, R² in the compound of Formula **I, II,** or **III,** or a pharmaceutically acceptable salt thereof is chloro, - SCH₃, -SOCH₃, -SO₂CH₃, or -O(CH₂)₂N(CH₃)₂, wherein the remaining variables are as described above for Formula **I.** In another alternative, as part of a fourth embodiment, R² in the compound of Formula **I, II,** or **III,** or a pharmaceutically acceptable salt thereof is chloro, wherein the remaining variables are as described above for Formula **I.**

In a fifth embodiment, R³ in the compound of Formula **I, II,** or **III,** or a pharmaceutically acceptable salt thereof is cyano, wherein the remaining variables are as described above for Formula **I** or the fourth embodiment.

In a sixth embodiment, R¹ in the compound of Formula **I, II,** or **III,** or a pharmaceutically acceptable salt thereof is hydrogen, fluoro, hydroxyl, or methyl, wherein the remaining variables are as described above for Formula **I** or the fourth or fifth embodiment. Alternatively, as part of a sixth embodiment, R¹ in the compound of Formula **I, II,** or **III,** or a pharmaceutically acceptable salt thereof is hydrogen, wherein the remaining variables are as described above for Formula **I** or the fourth or fifth embodiment.

In a seventh embodiment, R⁵ in the compound of Formula **I, II,** or **III,** or a pharmaceutically acceptable salt thereof is halo or cyano, wherein the remaining variables are as described above for Formula **I** or the fourth, fifth, or sixth embodiment. Alternatively, as part of a seventh embodiment, R⁵ in the compound of Formula **I, II,** or **III,** or a pharmaceutically acceptable salt thereof is halo, wherein the remaining variables are as described above for Formula **I** or the fourth, fifth, or sixth embodiment.

In an eighth embodiment, q in the compound of Formula **I, II,** or **III,** or a pharmaceutically acceptable salt thereof is 1, wherein the remaining variables are as described above for Formula **I** or the fourth, fifth, sixth, or seventh embodiment.

In a ninth embodiment, r in the compound of Formula **I, II,** or **III,** or a pharmaceutically acceptable salt thereof is 1, wherein the remaining variables are as described above for Formula **I** or the fourth, fifth, sixth, seventh, or eighth embodiment. Alternatively, as part of a ninth embodiment, r in the compound of Formula **I, II,** or **III,** or a pharmaceutically acceptable salt thereof is 0, wherein the remaining variables are as described above for Formula **I** or the fourth, fifth, sixth, seventh, or eighth embodiment.

In a tenth embodiment, R⁶ in the compound of Formula **I, II,** or **III,** or a pharmaceutically acceptable salt thereof is halo, wherein the remaining variables are as described above for Formula **I** or the fourth, fifth, sixth, seventh, or eighth embodiment.

In an eleventh embodiment, R⁷ in the compound of Formula **I, II,** or **III,** or a pharmaceutically acceptable salt thereof is halo, halo(C₁-C₄)alkyl, (C₁-C₄)alkyl, (C₁-C₄)alkoxy, -(C₁-C₄)alkylOR^{a}, -C(O)NR^{a}R^{b}, phenyl, 4- to 6-membered heterocyclyl, and 5- to 7-membered heteroaryl, wherein each of said phenyl, 4- to 6-membered heterocyclyl, and 5-to 7-membered heteroaryl are optionally and independently substituted with 1 to 3 groups selected from R⁸, wherein the remaining variables are as described above for Formula **I** or the fourth, fifth, sixth, seventh, eighth, ninth, or tenth embodiment. Alternatively, as part of an eleventh embodiment, R⁷ in the compound of Formula **I, II,** or **III,** or a pharmaceutically acceptable salt thereof is halo, halo(C₁-C₄)alkyl, (C₁-C₄)alkyl, (C₁-C₄)alkoxy, -(C₁-C₄)alkylOR^{a}, -C(O)NR^{a}R^{b}, phenyl, pyridinyl, piperazinyl, piperidinyl, pyrrolidinyl, thiomorpholinyl, pyrazolyl, and oxetanyl, wherein each of said phenyl, pyridinyl, pyrazolyl, pyrrolidinyl, piperazinyl, thiomorpholinyl, piperidinyl, and oxetanyl are optionally and independently substituted with 1 to 3 groups selected from R⁸, wherein the remaining variables are as described above for Formula **I** or the fourth, fifth, sixth, seventh, eighth, ninth, or tenth embodiment. In another alternative, as part of an eleventh embodiment, R⁷ in the compound of Formula **I, II,** or **III,** or a pharmaceutically acceptable salt thereof is halo, halo(C₁-C₄)alkyl, (C₁-C₄)alkyl, (C₁-C₄)alkoxy, -(C₁-C₄)alkylOR^{a}, -C(O)NR^{a}R^{b}, phenyl, pyridinyl, pyrazolyl, and oxetanyl, wherein each of said phenyl, pyridinyl, pyrazolyl, and oxetanyl are optionally and independently substituted with 1 to 3 groups selected from R⁸, wherein the remaining variables are as described above for Formula **I** or the fourth, fifth, sixth, seventh, eighth, ninth, or tenth embodiment.

In a twelfth embodiment, R⁸ in the compound of Formula **I, II,** or **III,** or a pharmaceutically acceptable salt thereof, is selected from halo, -C(O)NR^{d}R^{e}, (C₁-C₄)alkyl, halo(C₁-C₄)alkyl, (C₁-C₄)alkoxy, halo(C₁-C₄)alkoxy, oxo, and cyano, wherein the remaining variables are as described above for Formula **I** or third, fourth, fifth, sixth, seventh, eighth, ninth, tenth, or eleventh embodiment. Alternatively, as part of a twelfth embodiment, R⁸ in the compound of Formula **I, II,** or **III,** or a pharmaceutically acceptable salt thereof is selected from halo, (C₁-C₄)alkyl, halo(C₁-C₄)alkyl, (C₁-C₄)alkoxy, halo(C₁-C₄)alkoxy, oxo, and cyano, wherein the remaining variables are as described above for Formula **I** or third, fourth, fifth, sixth, seventh, eighth, ninth, tenth, or eleventh embodiment. In another alternative, as part of a twelfth embodiment, R⁸ in the compound of Formula **I, II,** or **III,** or a pharmaceutically acceptable salt thereof is selected from -C(O)NR^{d}R^{e}, (C₁-C₄)alkyl, and oxo, wherein the remaining variables are as described above for Formula **I** or third, fourth, fifth, sixth, seventh, eighth, ninth, tenth, or eleventh embodiment. In another alternative, as part of a twelfth embodiment, R⁸ in the compound of Formula **I, II,** or **III,** or a pharmaceutically acceptable salt thereof is selected from -C(O)N(CH₃)₂, CH₃, and oxo, wherein the remaining variables are as described above for Formula **I** or third, fourth, fifth, sixth, seventh, eighth, ninth, tenth, or eleventh embodiment. In another alternative, as part of a twelfth embodiment, R⁸ in the compound of Formula **I, II,** or **III,** or a pharmaceutically acceptable salt thereof is halo(C₁-C₄)alkyl, wherein the remaining variables are as described above for Formula **I** or third, fourth, fifth, sixth, seventh, eighth, ninth, tenth, or eleventh embodiment.

Compounds having the Formula **I, II,** or **III** are further disclosed in the Exemplification and are included in the present disclosure. Pharmaceutically acceptable salts thereof as well as the neutral forms are included.

### 4. Uses, Formulation and Administration

The compounds and compositions described herein are generally useful for modulating the activity of PPARG. In some aspects, the compounds, pharmaceutical acceptable salts, and pharmaceutical compositions described herein inhibit the activity PPARG. In some aspects, the compounds and pharmaceutical acceptable salts disclosed herein are agonists of PPARG. In some aspects, the compounds and pharmaceutical acceptable salts disclosed herein are agonists of PPARG. In some aspects, the compounds and pharmaceutical acceptable salts disclosed herein are inverse agonists of PPARG. In one aspect, "inverse-agonists" refer to agents that bind to the same receptor binding site as a agonist (e.g., the binding site of a nuclear receptor such as PPARG) and not only antagonizes the effects of an agonist but, moreover, exerts the opposite effect by suppressing spontaneous receptor signaling (when present).

In some aspects, the compounds and pharmaceutical acceptable salts disclosed herein overcome the activated state of PPARG function resulting from alteration in PPARG activity (mutation, amplification or overexpression) or from RXRA activating mutations. In some aspect, the compounds and pharmaceutical acceptable salts disclosed herein increase the repressive state (NCOR1 recruitment) to a higher degree than previously disclosed PPARG modulators such as prior inverse agonists. Such results even arise in the mutant context. See e.g., the table qualitatively assessing NCOR1 recruitment and repression of PPARG target genes in HT1197 in the Exemplification section.

In some aspects, the compounds and pharmaceutical compositions described herein are useful in treating a disorder associated with PPARG function. Thus, provided herein are methods of treating a disorder associated with PPARG function, comprising administering to a subject in need thereof, a therapeutically effective amount of a compound described herein, or a pharmaceutically acceptable salt thereof, or a pharmaceutical composition comprising a disclosed compound or pharmaceutically acceptable salt thereof.

Also provided is the use of a compound described herein, or a pharmaceutically acceptable salt thereof, or a pharmaceutical composition comprising a disclosed compound or pharmaceutically acceptable salt thereof, for the manufacture of a medicament for treating a disorder associated with PPARG function. Also provided is a compound described herein, or a pharmaceutically acceptable salt thereof, or a pharmaceutical composition comprising a disclosed compound or pharmaceutically acceptable salt thereof, for use in treating a disorder associated with PPARG.

In one aspect, the disorder associated with PPARG is cancer. In some aspects, the cancer is associated with an up-regulated peroxisome proliferator-activated receptor (PPAR) signaling pathway. In some aspects, the up-regulated PPAR signaling pathway is associated with increased expression of one or more genes selected from Uroplakin 1A (UPK1A), Uroplakin IB (UPK1B), Uroplakin (UPK2), Keratin 20 (KRT20), GATA Binding Protein 3 (GAT A3), Nuclear Receptor Corepressor 1 (NCORl), Nuclear Receptor Corepressor 2 (NCOR2), Fatty Acid Binding Protein 4 (FABP4), Forkhead Box Al (FOXA1), CD36 Molecule (CD36), Acyl-CoA Oxidase 1 (ACOX1), 3-Hydroxy-3-Methylglutaryl-CoA Synthase 2 (HMGCS2), Acyl-CoA Synthetase Long-Chain Family Member 5 (ACSL5), Arachidonate 5 -Lipoxygenase (ALOX5), Acyl-CoA Synthetase Long-Chain Family Member 1 (ACSL1), and Angiopoietin Like 4 (ANGPTL4).

In some aspects, the cancer treated by the compounds, pharmaceutically acceptable salt thereof, and pharmaceutical compositions described herein is selected from breast cancer, pancreatic cancer, ovarian cancer, prostate cancer, renal cancer, bladder cancer, testicular cancer, urothelial cancer (e.g., non-muscle-invasive urothelial cancer, muscle-invasive urothelial cancer, metastatic urothelial cancer), skin cancer, melanoma, colon cancer, kidney cancer, brain cancer and a hematopoietic cancer (e.g., lymphoma, multiple myeloma and leukemia). In one aspect, the cancer treated by the compounds, pharmaceutically acceptable salt thereof, and pharmaceutical compositions described herein is urothelial cancer such as non-muscle-invasive urothelial cancer, muscle-invasive urothelial cancer, and metastatic urothelial cancer.

Other uses besides cancer are contemplated and include e.g., metabolic diseases (e.g., osteoporosis, rachitis, arthrosis, obesity, type I and type II diabetes mellitus), lipid metabolism disorder, pancreatitis, glucose metabolism disorder, diabetic neuropathy, diabetic complications, hyperuricemia, osteoporosis, rachitis, arthrosis inflammatory diseases (e.g., inflammatory skin diseases such as psoriasis, atopic dermatitis, eczema, acne vulgaris, other dermatitides and pruritus), pulmonary disorders (e.g., asthma and chronic obstructive pulmonary disease), autoimmune disease, neurodegenerative disease (e.g., multiple sclerosis, Alzheimer's disease, and Parkinson's disease), cardiovascular diseases (e.g., selected from atherosclerosis, venous and arterial occlusive diseases), restenosis after invasive procedures, cardiomyopathy, myocardial fibrosis, congestive heart failure, angiogenesis and neovascularization in neoplastic diseases and renal diseases.

In certain aspects, a pharmaceutical composition described herein is formulated for administration to a patient in need of such composition. Pharmaceutical compositions described herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. In some embodiments, the compositions are administered orally, intraperitoneally or intravenously. Sterile injectable forms of the pharmaceutical compositions described herein may be aqueous or oleaginous suspension. These suspensions may be formulated according to techniques known in the art using suitable dispersing or wetting agents and suspending agents.

In some aspects, the pharmaceutical compositions are administered orally.

A specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound described herein in the composition will also depend upon the particular compound in the pharmaceutical composition.

### EXEMPLIFICATION

### Chemical Synthesis

The representative examples that follow are intended to help illustrate the present disclosure, and are not intended to, nor should they be construed to, limit the scope of the invention.

General starting materials used were obtained from commercial sources or prepared in other examples, unless otherwise noted.

### Preparation of Compounds

The compounds claimed herein were prepared following the procedures outlined in the following schemes.

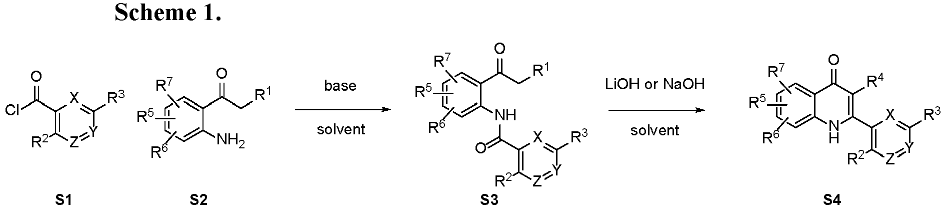

Quinolones like **S4** may be prepared by the general synthetic methods shown in Scheme 1. Compounds of formula **S3** may be prepared from the coupling an acid chloride **S1** with a 2-ketoaniline **S2** with or without an added base. Treatment of the amide **S3** with a base such as sodium hydroxide or lithium hydroxide at elevated temperature in an ethereal solvent such as dioxane or 2-methyl-tetrahydrofuran provides the target quinolone compound **S4.**

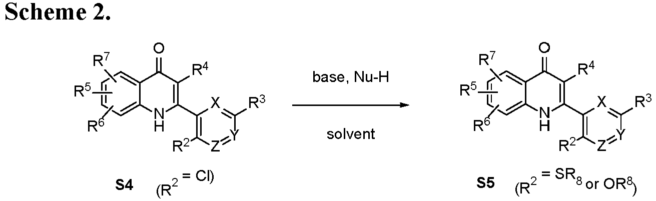

### Quinolones like S5 may be prepared by treating halo-quinolones like S4 with a base and nucleophile

### Preparation of Starting Materials

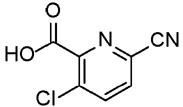

3-Chloro-6-cyanopicolinic acid: To a solution of ethyl 3-chloro-6-cyanopicolinate in DCE (10 mL) was added hydroxy(trimethyl)stannane (859 mg, 4.75 mmol, 2 equiv.) at 20°C. The mixture was stirred at 50°C for 16 hrs. The mixture was diluted with water (15 mL) and extracted with ethyl acetate (2 X 10 mL). The combined organic layers were washed with brine (15 mL X 2), dried with anhydrous Na₂SO₄, filtered, and concentrated under reduced pressure to afford the title compound (268 mg, 49% yield, 80% purity) as a white solid. ¹H NMR (400 MHz, CD₃OD) *δ* 8.20 (*d, J =* 8.6 Hz, 1H), 8.00 - 7.95 (m, 1H). LCMS [M+1] = 183.0/185.0.

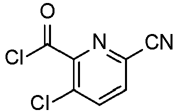

3-Chloro-6-cyanopicolinoyl chloride: 3-chloro-6-cyanopicolinic acid (120 mg, 657 µmol, 1 equiv.) was added to a flask containing SOCl₂ (2 mL) at 20°C. Then the mixture was stirred at 80°C for 3 hours under N₂. The reaction mixture was concentrated under reduced pressure to afford the title compound (130 mg, crude) as a yellow solid. The crude product was used directly in the next step without further purification.

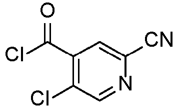

5-chloro-2-cyanoisonicotinoyl chloride: 5-chloro-2-cyanoisonicotinic acid (200 mg, 1.10 mmol, 1 equiv.) was added to a flask containing SOCl₂ (2 mL) at 20°C. Then the mixture was stirred at 80°C for 1 hour under N₂. The reaction mixture was concentrated under reduced pressure to afford the title compound (220 mg, crude) as white solid. The crude product was used directly in the next step without further purification.

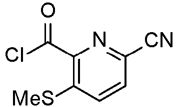

### 6-cyano-3-(methylthio)picolinoyl chloride

Step 1, 6-cyano-3-(methylthio)picolinic acid: To a solution of ethyl 3-chloro-6-cyanopicolinate (2 g, 9.5 mmol, 1.0 equiv.) in DMF (3 mL) was added sodium methanethiolate (1.5 g, 21.4 mmol, 2.2 equiv.). The mixture was stirred at 0 °C for 2 hours. The reaction mixture was poured into ice-water (15 mL) and stirred for 10 minutes. The pH of the solution was adjusted to pH = 5-6 with aqueous 1M HCl. The white solid that precipitated during the pH adjustment was filtered off and washed with H₂O (5 mL). The filter cake was dried under vacuum to afford the title compound (800 mg, 43% yield) as a white solid. ¹H NMR (400 MHz, DMSO-*d*₆) δ 8.13 - 8.08 (m, 1H), 8.05 - 7.99 (m, 1H), 2.49 (br s, 3H).

Step 2, 6-cyano-3-(methylthio)picolinoyl chloride: A solution of 6-cyano-3-(methylthio)picolinic acid (300 mg, 1.5 mmol, 1.0 equiv.) in SOCl₂ (5 mL) was stirred at 80 °C for 1 hour. The reaction mixture was concentrated under reduced pressure to afford the title compound (350 mg, crude) as a white solid. The product was used in next step without further purification.

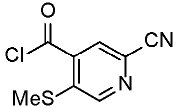

### 2-cyano-5-(methylthio)isonicotinoyl chloride

Step 1, 2-cyano-5-methylsulfanyl-pyridine-4-carboxylic acid: To a solution of sodium methanethiolate (17.83 g, 254 mmol, 2.5 equiv.) in DMF (130 mL) was added a solution of methyl 5-chloro-2-cyano-pyridine-4-carboxylate (20 g, 102 mmol, 1.0 equiv.) in DMF (260 mL) at 0 °C. The mixture was stirred at 0 °C for 0.5 hours under N₂. The pH of the reaction mixture was adjusted to pH = 1 with concentrated HCl. The white solid that precipitated during the pH adjustment was filtered off and washed with H₂O (5 mL). The filter cake was dried under vacuum to afford the title compound (18.0 g, 91% yield) as a white solid. ¹H NMR (400 MHz, DMSO-*d*₆) δ 8.75 (s, 1H), 8.21 (s, 1H), 2.62 (s, 3H).

Step 2, 2-cyano-5-(methylthio)isonicotinoyl chloride: A solution of 2-cyano-5-(methylthio)isonicotinic acid (115 mg, 592 µmol, 1.0 equiv.) in SOCl₂ (4 mL) was stirred at 100 °C for 2 hours under N₂. The mixture was concentrated in vacuum to afford the title compound (125 mg, crude) as a yellow solid. The product was used in next step without further purification.

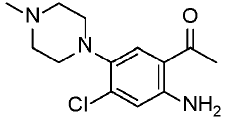

### 1-(2-amino-4-chloro-5-(4-methylpiperazin-1-yl)phenyl)ethanone

Step 1, 1-(5-bromo-2-chloro-4-nitro-phenyl)-4-methyl-piperazine: To a solution of 1-bromo-4-chloro-5-fluoro-2-nitro-benzene (2.0 g, 7.86 mmol, 1.0 equiv.) in DMF (20 mL) was added 1-methylpiperazine (787 mg, 7.86 mmol, 1.0 equiv.) and K₂CO₃ (1.09 g, 7.86 mmol, 1.0 equiv.). The mixture was stirred at RT for 4 hours. The mixture was added to water (100 mL). The precipitate that formed was filtered off and the material was washed with water (3 x 50 mL). The material was purified by silica gel column chromatography (10:1 to 3:1 petroleum ether:ethyl acetate + 10% DCM) to afford the title compound (2.4 g, 91% yield) as an orange solid. LCMS: [M+1] = 334.0. ¹H NMR (400 MHz, CHLOROFORM-*d*) δ 8.05 (s, 1H), 7.24 (s, 1H), 3.29 - 3.17 (m, 4H), 2.68 - 2.55 (m, 4H), 2.38 (s, 3H).

Step 2, 2-bromo-5-chloro-4-(4-methylpiperazin-1-yl)aniline: A mixture of 1-(5-bromo-2-chloro-4-nitro-phenyl)-4-methyl-piperazine (2.4 g, 7.17 mmol, 1.0 equiv.), Fe (2.00 g, 35.8 mmol, 5.0 equiv.) and NH₄Cl (1.92 g, 35.8 mmol, 5.0 equiv.) in MeOH (20 mL) and H₂O (5 mL) was purged with N₂ and then the mixture was stirred at 80 °C for 16 hours under N₂. The suspension was filtered through a pad of Celite and the pad cake was washed with MeOH (3 x 30 mL). The filtrate was concentrated under reduced pressure. The residue was diluted with water (20 mL). The pH of the solution was adjusted pH = 7-8 with saturated aqueous sodium bicarbonate solution. The aqueous phase was extracted with ethyl acetate (3 x 30 mL). The combined organic layers were washed with brine (10 mL), dried over Na₂SO₄, filtered, and concentrated under reduced pressure to afford the title compound (1.7 g, crude) as an orange solid. LCMS: [M+1] = 304.0. ¹H NMR (400 MHz, DMSO-*d*₆) δ 7.11 (s, 1H), 6.87 (s, 1H), 5.29 - 5.16 (m, 2H), 2.80 (br t, *J =* 4.4 Hz, 4H), 2.42 (br s, 4H), 2.20 (s, 3H).

Step 3, 5-chloro-2-(1-ethoxyvinyl)-4-(4-methylpiperazin-1-yl)aniline: To a solution of 2-bromo-5-chloro-4-(4-methylpiperazin-1-yl)aniline (300 mg, 985 µmol, 1 equiv.) and tributyl(1-ethoxyvinyl)stannane (399 uL, 1.18 mmol, 1.2 equiv.) in toluene (5 mL) was added Pd(PPh₃)₄ (113.8 mg, 98.4 µmol, 0.1 equiv.). The mixture was stirred at 120 °C for 16 hours under N₂. The reaction mixture was cooled to RT and quenched with an aqueous solution of KF (20 mL). The aqueous phase was extracted with ethyl acetate (2 x 30 mL) and the combined organic layers were washed with saturated aqueous sodium bicarbonate, brine, dried with anhydrous Na₂SO₄, filtered, and concentrated to afford the title compound (250 mg, crude) as brown solid. LCMS [M+1] = 296.1. ¹H NMR (400 MHz, CHLOROFORM-d) δ 7.39 (s, 1H), 6.74 (s, 1H), 6.11 (br s, 2H), 3.00 (br s, 4H), 2.62 (br s, 4H), 2.59 - 2.52 (m, 3H), 2.41 - 2.31 (m, 3H), 1.30 - 1.23 (m, 2H).

Step 4, 1-(2-amino-4-chloro-5-(4-methylpiperazin-1-yl)phenyl)ethanone: A solution of 5-chloro-2-(1-ethoxyvinyl)-4-(4-methylpiperazin-1-yl)aniline (250 mg, 845 µmol, 1.0 equiv.) in aqueous HCl (1 M, 2.54 mL, 3.0 equiv.) was stirred at 20 °C for 2 hours. The reaction mixture was diluted with H₂O (10 mL) and extracted with EtOAc (3 x 10 mL). The pH of the aqueous layer was adjusted to pH = 8 with saturated aqueous NaHCO₃. The aqueous layer was extracted with ethyl acetate (3 x 10 mL). The combined organic layers were dried over Na₂SO₄, filtered, and concentrated under reduced pressure. The resulting residue was purified by silica gel column chromatography (50:1 to 10:1 DCM:MeOH) to afford the title compound (140 mg, 62% yield) as a yellow solid. ¹H NMR (400 MHz, CHLOROFORM-*d*) δ 7.39 (s, 1H), 6.74 (s, 1H), 6.11 (br s, 2H), 3.00 (br s, 4H), 2.69 - 2.57 (m, 4H), 2.57 - 2.54 (m, 3H), 2.43 - 2.31 (m, 3H).

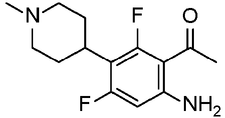

### 1-(6-amino-2,4-difluoro-3-(1-methylpiperidin-4-yl)phenyl)ethan-1-one

Step 1, 1-(6-amino-2,4-difluoro-3-(1-methyl-1,2,3,6-tetrahydropyridin-4-yl)phenyl)ethan-1-one: To a mixture of 1-(6-amino-3-bromo-2,4-difluorophenyl)ethan-1-one (1.3 g, 5.3 mmol, 1 equiv.) and 1-methyl-4-(4,4,5,5-tetramethyl-1,3,2-dioxaborolan-2-yl)-3,6-dihydro-2H-pyridine (1.8 g, 8.0 mmol, 1.5 equiv.) in THF (1.2 mL) and H₂O (0.3 mL) was added K₃PO₄ (2.5 g, 11.7 mmol, 2.2 equiv.) and di-*tert*-butyl(cyclopentyl)phosphane-dichloropalladium;iron (347 mg, 532 µmol, 0.1 equiv.) at RT under N₂. The mixture was stirred at 80 °C for 2 hours. The reaction mixture was filtered and concentrated under reduced pressure. The residue was purified by silica column chromatography (10:1 to 2:1 petroleum ether:ethyl acetate) to afford the title compound (1.4 g, 97% yield) as a yellow oil. ¹H NMR (400 MHz, METHANOL-*d*₄) δ 6.29 (dd, *J* = 1.8, 12.2 Hz, 1H), 5.73 (br s, 1H), 3.13 (q, *J =* 2.8 Hz, 2H), 2.70 (t, *J =* 5.8 Hz, 2H), 2.51 (d, *J =* 8.8 Hz, 3H), 2.43 (br d, *J* = 1.8 Hz, 2H), 2.38 (s, 3H).

Step 2, 1-(6-amino-2,4-difluoro-3-(1-methylpiperidin-4-yl)phenyl)ethan-1-one: To a solution of 1-(6-amino-2,4-difluoro-3-(1-methyl-1,2,3,6-tetrahydropyridin-4-yl)phenyl)ethan-1-one (1.2 g, 4.4 mmol, 1 equiv.) in MeOH (12 mL) was added 20% Pd(OH)₂ on carbon (500 mg) under N₂. The suspension was degassed under vacuum and purged with H₂ several times. The mixture was stirred under H₂ (50 Psi) at 70 °C for 3 hours. The reaction mixture was filtered and concentrated under reduced pressure to afford the title compound (1.0 g, 84% yield) as a light-yellow solid. ¹H NMR (400 MHz, METHANOL-*d*₄) δ 6.27 (dd, *J =* 1.8, 13.2 Hz, 1H), 2.99 (br d, *J =* 6.8 Hz, 2H), 2.92 - 2.83 (m, 1H), 2.51 (d, *J =* 9.2 Hz, 3H), 2.30 (s, 3H), 2.19 - 2.07 (m, 4H), 1.72 - 1.65 (m, 2H).

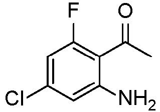

1-(2-amino-4-chloro-6-fluorophenyl)ethanone: To a solution of 3-chloro-5-fluoroaniline (20 g, 137 mmol, 1.0 equiv.) in *p*-xylene (40 mL) was added BCl₃ (1 M, 182.7 mL, 1.3 equiv.) at 0-5 °C over 2 hours. The mixture was warmed up to RT within 0.5 hours and stirred at RT for 10 minutes. Then acetonitrile (57.8 mL, 1.10 mol, 8 equiv.) was added dropwise at RT over 20 minutes. The mixture was stirred at RT for 10 minutes, then*p-*xylene (45 mL) was added. Then AlCl₃ (10.2 g, 76.9 mmol, 0.5 equiv.) was added and the reaction mixture was stirred at RT for 1 hour and then stirred at 75-77 °C for another 12 hours. Then aqueous HCl (4 N, 200 mL) was added to the mixture and the mixture was stirred at 80 °C for 4 hours. The mixture was poured into water (800 mL) and extracted with ethyl acetate (1.5 L). The organic phase was washed with brine (100 mL), dried with anhydrous Na₂SO₄, filtered, and concentrated under reduced pressure. The residue was purified by silica gel column chromatography (50:1 to 20:1 petroleum ether:ethyl acetate) to afford the title compound (4.6 g, 18% yield) as a light-yellow solid. LCMS [M+1] = 188.0/190.0. ¹H NMR (400 MHz, CHLOROFORM-*d*) δ 6.53 - 6.41 (m, 2H), 6.36 (dd, *J* = 2.0, 11.8 Hz, 2H), 2.58 (d, *J* = 8.4 Hz, 3H).

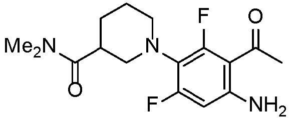

1-(3-acetyl-4-amino-2,6-difluorophenyl)- N,N-dimethylpiperidine-3-carboxamide: The title compound was prepared following the synthetic steps described for 1-(2-amino-4-chloro-5-(4-methylpiperazin-1-yl)phenyl)ethenone using 2-bromo-3,4,5-trifluoro-1-nitrobenzene and N,N-dimethylpiperidine-3-carboxamide as the starting materials. LCMS [M-1] = 324.1. ¹H NMR (400 MHz, CHLOROFORM-*d*) δ 6.41 - 6.18 (m, 2H), 6.12 (dd, *J =* 1.6, 12.6 Hz, 1H), 3.34 - 3.20 (m, 1H), 3.10 (s, 3H), 3.03 (br d, *J =* 16.0 Hz, 2H), 2.94 (s, 3H), 2.57 (d, *J =* 8.8 Hz, 3H), 1.95 - 1.84 (m, 1H), 1.82 - 1.53 (m, 5H).

### Example 1

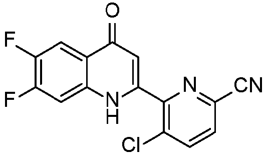

### 5-chloro-6-(6,7-difluoro-4-oxo-1,4-dihydroquinolin-2-yl)picolinonitrile

Scheme 1, step 1. N-(2-acetyl-4,5-difluorophenyl)-3-chloro-6-cyanopicolinamide: To a solution of 1-(2-amino-4,5-difluorophenyl)ethan-1-one (100 mg, 584 µmol, 1 equiv.) in THF (2 mL) was added NaH (25.7 mg, 642 µmol, 60% purity, 1.1 equiv.) at 0°C. The mixture was stirred at 0°C for 5 min under N₂. Then a solution of 3-chloro-6-cyanopicolinoyl chloride (129 mg, 642 µmol, 1 equiv.) in THF (1 mL) was added dropwise to the mixture at 0°C and the mixture was stirred at 20°C for 16 hours. The reaction mixture was quenched by the addition of saturated aqueous NH₄Cl (20 mL), and the mixture was extracted with ethyl acetate (3 X 20 mL). The combined organic layers were washed with brine, dried over Na₂SO₄, filtered, and concentrated under reduced pressure. The crude product was triturated with ethyl acetate (3 mL) at 20°C for 20 min. Then the product was then filtered off and the precipitate was washed with ethyl acetate (3 X 0.3 mL). The solid was dried under reduced pressure to afford the title compound (130 mg, 66% yield) as a white solid. ¹H NMR (400 MHz, DMSO-*d*₆) δ 13.03 (s, 1H), 8.68 (dd, *J* = 7.8, 13.8 Hz, 1H), 8.45 (d, *J =* 8.4 Hz, 1H), 8.37 - 8.21 (m, 2H), 2.75 - 2.64 (m, 3H).

Scheme 1, step 2. 5-chloro-6-(6,7-difluoro-4-oxo-1,4-dihydroquinolin-2-yl)picolinonitrile: To a solution of N-(2-acetyl-4,5-difluorophenyl)-3-chloro-6-cyanopicolinamide (100 mg, 298 µmol, 1 equiv.) in dioxane (5 mL) was added LiOH (7.1 mg, 297 µmol, 1 equiv.) at 20°C. The mixture was stirred at 110°C for 16 hours under N₂. The pH of the mixture was adjusted to 6 with aqueous 1 M HCl. Then the mixture was diluted with water (15 mL) and extracted with ethyl acetate (3 X 20 mL). The combined organic layers were washed with brine, dried over Na₂SO₄, filtered, and concentrated under reduced pressure. The residue was purified by preparative-HPLC (Column: Waters Xbridge BEH C18 100mm X 30mm X 10um; mobile phase: 10-40% acetonitrile in water (+NH₄HCO₃)) to afford the title compound (21 mg, 21% yield) as a yellow solid. ¹H NMR (400 MHz, DMSO-*d*₆) δ 8.49 (d, *J* = 8.4 Hz, 1H), 8.28 (d, *J* = 8.4 Hz, 1H), 8.00 (dd, *J* = 9.0, 11.0 Hz, 1H), 7.72 (dd, *J* = 7.0, 11.4 Hz, 1H), 6.53 (s, 1H). LCMS [M+1] = 318.0/320.0.

### Example 2

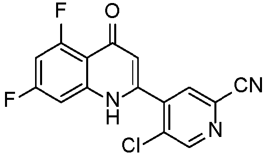

### 5-chloro-4-(5,7-difluoro-4-oxo-1,4-dihydroquinolin-2-yl)picolinonitrile

Scheme 1, step 1. N-(2-acetyl-3,5-difluorophenyl)-5-chloro-2-cyanoisonicotinamide: To a solution of 1-(2-amino-4,6-difluorophenyl)ethan-1-one (170 mg, 993 µmol, 1 equiv.) in THF (1 mL) was added NaH (43.7 mg, 1.09 mmol, 60% purity, 1.1 equiv.) at 0°C. Then a solution of 5-chloro-2-cyanoisonicotinoyl chloride (220 mg, 1.09 mmol, 1.1 equiv.) in THF (1 mL) was added dropwise to the mixture. The mixture was stirred at 20°C for 16 hr. The reaction mixture was quenched by the addition of saturated aqueous NH₄Cl (15 mL) and then extracted with ethyl acetate (2 X 30 mL). The combined organic layers were washed with brine (30 mL), dried over anhydrous Na₂SO₄, filtered, and concentrated under reduced pressure. The resulting residue was purified by preparative TLC (petroleum ether:ethyl acetate = 3:1) to afford the title compound (84 mg, 25% yield) as a yellow solid. ¹H NMR (400 MHz, CDCl₃) δ 12.56 (br s, 1H), 8.84 (s, 1H), 8.52 - 8.45 (m, 1H), 7.91 (s, 1H), 6.77 - 6.74 (dd, *J* = 2.6, 8.2 Hz, 1H), 2.70 (d, *J* = 8.4 Hz, 3H).

Scheme 1, step 2. 5-chloro-4-(5,7-difluoro-4-oxo-1,4-dihydroquinolin-2-yl)picolinonitrile: To a solution of N-(2-acetyl-3,5-difluorophenyl)-5-chloro-2-cyanoisonicotinamide (53 mg, 158 µmol, 1 equiv.) in dioxane (1 mL) was added LiOH (7.6 mg, 316 µmol, 2 equiv.) at 20°C under N₂. The mixture was stirred at 110°C for 1 hr. The reaction mixture was concentrated under reduced pressure and the pH was adjusted to 6-7 with aqueous 1M HCl. The mixture was filtered. Then the filtrate was washed with water and dried under reduced pressure to afford the title compound (31 mg, 58% yield) as a white solid. ¹H NMR (400 MHz, DMSO-d₆) δ 12.23 (br s, 1H), 9.10 (s, 1H), 8.49 (s, 1H), 7.24 - 7.10 (m, 2H), 6.18 (s, 1H). LCMS [M+1] = 318.0/320.0.

### Example 3

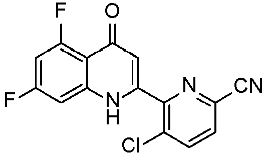

### 5-chloro-6-(5,7-difluoro-4-oxo-1,4-dihydroquinolin-2-yl)picolinonitrile

Scheme 1, step 1. N-(2-acetyl-3,5-difluorophenyl)-3-chloro-6-cyanopicolinamide: NaH (20.6 mg, 514 µmol, 60% purity, 1.1 equiv.) was added to a solution of 1-(2-amino-4,6-difluorophenyl)ethan-1-one in THF (0.8 mL) at 0°C. Then a solution of 3-chloro-6-cyanopicolinoyl chloride (1.1 equiv.) in THF (0.4 mL) was added to the solution at 0°C. The mixture was stirred at 20°C for 16 h. The mixture was diluted with water (15 mL) and extracted with ethyl acetate (2 X 10 mL). The combined organic layers were washed with brine (2 X 15 mL), dried with anhydrous Na₂SO₄, filtered, and concentrated under reduced pressure. The crude product was triturated with ethyl acetate (1.5 mL) at 20°C to afford the title compound (102 mg, 57% yield, 87% purity) as a white solid. ¹H NMR (400 MHz, DMSO-*d*₆) *δ* 12.38 (s, 1H), 8.45 (*d*, *J =* 8.4 Hz, 1H), 8.33 (*d*, *J =* 8.4 Hz, 1H), 8.06 - 8.01 (m, 1H), 7.30 (*ddd*, *J =* 2.4, 9.0, 11.6 Hz, 1H), 2.61 (*d*, *J* = 6.4 Hz, 3H). LCMS [M+1] = 336.0/338.0.

Scheme 1, step 2. 5-chloro-6-(5,7-difluoro-4-oxo-1,4-dihydroquinolin-2-yl)picolinonitrile: To a solution of N-(2-acetyl-3,5-difluorophenyl)-3-chloro-6-cyanopicolinamide in dioxane (1 mL) was added LiOH (5.0 mg, 209 µmol, 1 equiv.) at 20°C. The mixture was stirred at 110°C for 2 hrs. Then an additional portion of LiOH (5.0 mg, 209 µmol, 1 equiv.) was added to the reaction mixture at 20°C and the mixture was stirred at 110°C for another 2 h. The pH of the reaction mixture was adjusted to 5-6 with aqueous 1M HCl. The mixture was extracted with ethyl acetate (3 X 10 mL). The combined organic layers were washed with brine (20 mL), dried over Na₂SO₄, filtered and concentrated. The residue was purified by preparative HPLC (Column: Phenomenex Luna 80mm X 30mm X 3um; Mobile phase: water (HCl)-acetonitrile) to afford the title compound (28.6 mg, 43% yield) as a white solid. ¹H NMR (400 MHz, CD₃OD) *δ* 8.35 (d, *J* = 8.6 Hz, 1H), 8.08 (s, 1H), 7.29 (br *d*, *J =* 9.6 Hz, 1H), 7.00-7.11 (m, 1H), 6.75 ppm (s, 1H). LCMS [M+1] = 317.9/319.9.

The compounds in Table **1** were prepared following Scheme 1 using similar procedures to those described for Examples **1, 2,** and **3.**

**Table 1**

| Example number | Structure | IUPAC Name | ¹H NMR | LCMS Exact Mass |
|---|---|---|---|---|
| **4** | 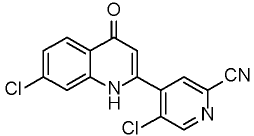 | 5-chloro-4-(7-chloro-4-oxo-1,4-dihydroquinolin-2-yl)picolinonitrile | (400 MHz, DMSO-*d₆*) δ 12.32 - 12.10 (m, 1H), 9.09 (s, 1H), 8.48 (s, 1H), 8.14 *(d, J = 8.6 Hz,* 1H), 7.65 (br s, 1H), 7.45 (br d, *J =* 7.8 Hz, 1H), 6.50 - 6.27 (m, 1H). | Calculated *m*/*z=*316.0, found *m*/*z* =315.9 |

### Example 5

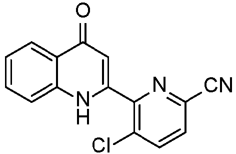

### 5-chloro-6-(4-oxo-1,4-dihydroquinolin-2-yl)picolinonitrile

Scheme 1, step 1. N-(2-acetylphenyl)-3-chloro-6-cyanopicolinamide: To a solution of 1-(2-aminophenyl)ethan-1-one (200 mg, 1.48 mmol, 1 equiv.) in DCM (2.0 mL) was added triethylamine (618 µL, 4.44 mmol, 3 equiv.). Then a solution of 3-chloro-6-cyanopicolinoyl chloride (360 mg) in DCM (2.0 mL) was added to the mixture dropwise at 25°C. The mixture was stirred at 25 °C for 0.5 h. The reaction mixture was partitioned between water (5 mL) and DCM (15 mL). The organic phase was separated, dried over Na₂SO₄, filtered, and concentrated under reduced pressure. The resulting residue was triturated with methanol (3 mL) and dried under reduced pressure to afford the title compound (140 mg, 32% yield) as a white solid. ¹H NMR (400 MHz, DMSO-*d*₆) δ 12.85 (br s, 1H), 8.75 - 8.61 (m, 1H), 8.50 - 8.41 (m, 1H), 8.32 - 8.11 (m, 2H), 7.77 - 7.63 (m, 1H), 7.36 - 7.26 (m, 1H), 2.75 - 2.67 (m, 3H). LCMS [M+1] = 300.1/302.1.

Scheme 1, step 2. 5-Chloro-6-(4-oxo-1,4-dihydroquinolin-2-yl)picolinonitrile: To a solution of LiOH (12.8 mg, 534 µmol, 2 equiv.) in dioxane (1.00 mL) was added N-(2-acetylphenyl)-3-chloro-6-cyanopicolinamide (80 mg, 267 µmol, 1 equiv.) at 25°C. The mixture was stirred at 110 °C for 6 hrs. The reaction mixture was quenched by addition of saturated aqueous NH₄Cl solution (3.00 mL), and the mixture was extracted with ethyl acetate (3 X 5 mL). The combined organic layers were dried over Na₂SO₄, filtered, and concentrated under reduced pressure. The residue was purified by preparative HPLC (Column: Phenomenex luna C18 80mm X 40mm X 3 µm; Mobile phase: 20-40% acetonitrile in water (+HCl)) to afford the title compound (20 mg, 27% yield) as a white solid. ¹H NMR (400 MHz, CD₃OD) δ 8.49 (*d, J =* 8.4 Hz, 1H), 8.44 (*d, J =* 8.4 Hz, 1H), 8.17 (*d, J =* 8.4 Hz, 1H), 8.08 (*d, J =* 3.6 Hz, 2H), 7.82 (td, *J =* 4.2, 8.4 Hz, 1H), 7.44 - 7.41 (m, 1H). LCMS [M+1] = 281.9/284.0.

The compounds in Table **2** were prepared following Scheme 1 using similar procedures to those described for Example **5.**

**Table 2**

| Example number | Structure | IUPAC Name | ¹H NMR | LCMS Exact Mass |
|---|---|---|---|---|
| **6** | 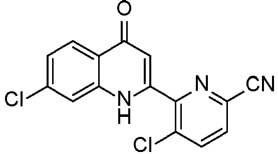 | 5-chloro-6-(7-chloro-4-oxo-1,4-dihydroquinolin-2-yl)picolinonitrile | (400 MHz, DMSO-*d₆*) δ 8.53 (d, *J* = 8.2 Hz, 1H), 8.32 (d, *J =* 8.4 Hz, 1H), 8.15 (d, *J =* 8.6 Hz, 1H), 7.78 (d, *J* = 1.6 Hz, 1H), 7.45 (dd, *J =* 1.8, 8.6 Hz, 1H), 6.51 (br s, 1H). | Calculated *m*/*z*=316.0, found *m*/*z* =315.9 |

### Example 7

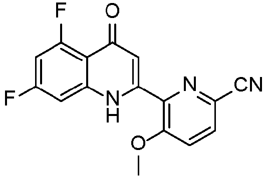

### 6-(5,7-difluoro-4-oxo-1,4-dihydroquinolin-2-yl)-5-methoxypicolinonitrile

Scheme 2, step 1. 6-(5,7-difluoro-4-oxo-1,4-dihydroquinolin-2-yl)-5-methoxypicolinonitrile: NaOMe (85.0 mg, 1.5 mmol, 5 equiv.) was added to a solution of 5-chloro-6-(5,7-difluoro-4-oxo-1,4-dihydroquinolin-2-yl)picolinonitrile (100 mg, 315 µmol, 1 equiv.) in DMF (1 mL) cooled to 0°C. The reaction mixture was then stirred for 16 hours at 20 °C. The mixture was concentrated in vacuum and the residue was purified by preparative HPLC (column: Phenomenex Luna C18 75mm X 30mm X 3um; mobile phase: 40-70% acetonitrile in water (+ formic acid)) to afford the title compound (20 mg, 20% yield) as a white solid. ¹H NMR (400 MHz, DMSO-*d₆*) δ 11.77 (br s, 1H), 8.26 (d, *J =* 8.8 Hz, 1H), 7.93 (d, *J =* 8.8 Hz, 1H), 7.50 (br d, *J* = 10.4 Hz, 1H), 7.10 (br t, *J =* 9.8 Hz, 1H), 6.68 (s, 1H), 4.04 (s, 3H). LCMS [M+1] = 314.0.

### Example 8

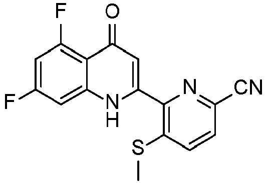

### 6-(5,7-difluoro-4-oxo-1,4-dihydroquinolin-2-yl)-5-(methylthio)picolinonitrile

Scheme 2, step 1. 6-(5,7-difluoro-4-oxo-1,4-dihydroquinolin-2-yl)-5-(methylthio)picolinonitrile: NaSMe (56.0 mg, 799 µmol, 2.5 equiv.) was added to a solution of 5-chloro-6-(5,7-difluoro-4-oxo-1,4-dihydroquinolin-2-yl)picolinonitrile (100 mg, 315 µmol, 1 equiv.) in DMF (1 mL) cooled to 0°C and the mixture was stirred for 1 h at 0°C. The reaction mixture was concentrated under reduced pressure and the resulting residue was purified by preparative HPLC (column: Phenomenex Luna C18 75mm X 30mm X 3um; mobile phase: 20%-60% acetonitrile in water (+ formic acid)) to afford the title compound (57 mg, 55% yield) as a white solid. ¹H NMR (400 MHz, CD₃OD) δ 8.04 (d, *J =* 8.6 Hz, 1H), 7.97 - 7.91 (m, 1H), 7.24 (br d, *J =* 9.4 Hz, 1H), 6.99 (ddd, *J =* 2.4, 9.4, 11.6 Hz, 1H), 6.63 (br s, 1H), 2.60 (s, 3H). LCMS [M+1] = 330.0.

### Example 9

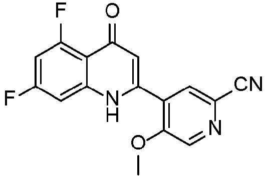

### 4-(5,7-difluoro-4-oxo-1,4-dihydroquinolin-2-yl)-5-methoxypicolinonitrile

Scheme 2, step 1. 4-(5,7-difluoro-4-oxo-1,4-dihydroquinolin-2-yl)-5-methoxypicolinonitrile: To a solution of 5-chloro-4-(5,7-difluoro-4-oxo-1,4-dihydroquinolin-2-yl)picolinonitrile_(100 mg, 315 µmol, 1 equiv.) in DMF (1.5 mL) was added NaOMe (85.0 mg, 1.5 mmol, 5 equiv.) at 0°C. The mixture was stirred at 20°C for 16 hrs. The reaction solution was concentrated in vacuum. The residue was purified by preparative HPLC (column: Phenomenex Luna 80mm X 30mm X 3um; mobile phase: 15-45% methanol in water (+HCl)) to afford the title compound (4.6 mg, 4.5% yield) as a yellow solid. ¹H NMR (400 MHz, DMSO-*d*₆): *δ* 8.79 (s, 1H), 8.25-8.29 (m, 1H), 7.11-7.25 (m, 2H), 6.24-6.37 (m, 1H), 4.08 (s, 3H). LCMS [M+1] = 314.0.

### Example 10

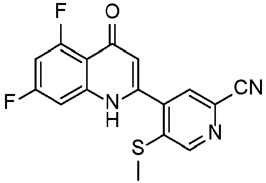

### 4-(5,7-difluoro-4-oxo-1,4-dihydroquinolin-2-yl)-5-(methylthio)picolinonitrile

Scheme 2, step 1. 4-(5,7-difluoro-4-oxo-1,4-dihydroquinolin-2-yl)-5-(methylthio)picolinonitrile: To a solution of 5-chloro-4-(5,7-difluoro-4-oxo-1,4-dihydroquinolin-2-yl)picolinonitrile (80 mg, 252 µmol, 1 equiv.) in DMF (1.5 mL) was added NaSMe (44.8 mg, 640 µmol, 2.54 equiv.) at 0°C. The mixture was stirred at 0°C for 1 hr. The reaction mixture was concentrated under reduced pressure. The residue was purified by preparative HPLC (column: Phenomenex Luna C18 75mm X 30mm X 3um; mobile phase: 30-60% acetonitrile in water (+formic acid)) to afford the title compound (16 mg, 19% yield) as a white solid. ¹H NMR (400 MHz, DMSO-*d₆*): *δ* 11.96-12.26 (m, 1H), 8.81 (s, 1H), 8.19 (s, 1H), 7.11-7.25 (m, 2H), 6.11-6.26 (m, 1H), 2.66-2.66 (m, 3H). LCMS [M+1] = 329.9.

The compound in Table 3 was prepared following Scheme 1 using similar procedures to those described for Example 7.

**Table 3**

| Example number | Structure | IUPAC Name | ¹H NMR | LCMS Exact Mass |
|---|---|---|---|---|
| **11** | 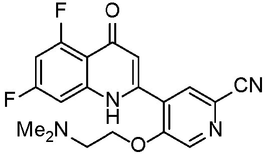 | 4-(5,7-difluoro-4-oxo-1,4-dihydroquinolin-2-yl)-5-(2-(dimethylamino)-ethoxy)-picolinonitrile | (400 MHz, METHANOL-*d*₄) *δ* 8.75 (s, 1H), 8.17 (s, 1H), 7.21 (br d, *J =* 9.2 Hz, 1H), 7.02 (ddd, *J =* 2.4, 9.4, 11.8 Hz, 1H), 6.47 (s, 1H), 4.77 - 4.72 (m, 2H), 3.69 - 3.64 (m, 2H), 2.91 (s, 6H) | Calculated *m*/*z*=371.1, found *m*/*z* =371.0 |

### Example 12

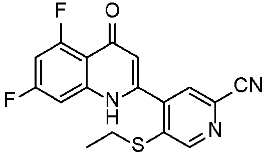

### 4-(5,7-difluoro-4-oxo-1,4-dihydroquinolin-2-yl)-5-(ethylthio)picolinonitrile

Scheme 2, step 1. 4-(5,7-difluoro-4-oxo-1,4-dihydroquinolin-2-yl)-5-(ethylthio)picolinonitrile: To a solution of ethanethiol (29.1 µL, 393 µmol, 2.5 equiv.) in DMF (2 mL) was added NaH (15.7 mg, 393 µmol, 2.5 equiv.; 60% dispersion in oil) at 0 °C and the mixture was stirred for 30 minutes under N₂. Then 5-chloro-4-(5,7-difluoro-4-oxo-1H-quinolin-2-yl) pyridine-2-carbonitrile (50 mg, 157 µmol, 1 equiv.) was added to the mixture in one portion. The mixture was stirred at RT for 16 hours under N₂. The reaction mixture was poured into saturated aqueous NH₄Cl (5mL) and stirred for 2 minutes. The aqueous phase was extracted with ethyl acetate (2 x 5 mL). The combined organic layers were washed with brine (2 x 5 mL), dried with anhydrous Na₂SO₄, filtered, and concentrated under reduced pressure. The residue was purified by preparative HPLC (column: Phenomenex Luna C18 75 x 30mm x 3um; mobile phase: 1-40% acetonitrile in water (+formic acid modifier) to afford the title compound (51.7 mg, 91% yield) as a white solid. LCMS: [M+1] = 344.0. ¹H NMR (400 MHz, DMSO-*d*₆) δ 12.11 (br s, 1 H) 8.85 (s, 1 H) 8.21 (s, 1 H) 7.05 - 7.24 (m, 2 H) 6.06 (br s, 1 H) 3.23 (br d, *J* = 7.2 Hz, 2 H) 1.26 (t, *J =* 7.2 Hz, 3 H).

### Example 13

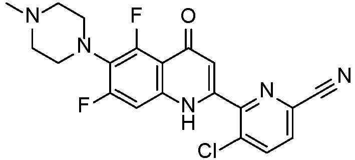

### 5-chloro-6-(5,7-difluoro-6-(4-methylpiperazin-1-yl)-4-oxo-1,4-dihydroquinolin-2-yl)picolinonitrile

Step 1, 1-(3-bromo-2,6-difluoro-4-nitro-phenyl)-4-methyl-piperazine: To a solution of 1-methylpiperazine (1.73 mL, 15.6 mmol, 1.0 equiv.) and K₂CO₃ (3.24 g, 23.4 mmol, 1.5 equiv.) in DMSO (40 mL) was added 4-bromo-1,2,3-trifluoro-5-nitro-benzene (4 g, 15.63 mmol, 1.0 equiv.). The mixture was stirred at 20 °C for 16 hours. The reaction mixture was diluted with water (100 mL) and extracted with EtOAc (3 x 50 mL). The combined organic layers were washed with brine (2 x 100 mL), dried over Na₂SO₄, filtered, and concentrated under reduced pressure to afford the title compound (4.73 g, 90% yield) as a brown solid. LCMS [M+1] = 336.0. ¹H NMR (400 MHz, CHLOROFORM-*d*) δ 7.66 (dd, *J =* 1.8, 11.8 Hz, 1H), 3.40 (br t, *J =* 4.6 Hz, 4H), 2.58 - 2.51 (m, 4H), 2.36 (s, 3H).

Step 2, 2-bromo-3,5-difluoro-4-(4-methylpiperazin-1-yl)aniline: A solution of 1-(3-bromo-2,6-difluoro-4-nitro-phenyl)-4-methyl-piperazine (2.5 g, 7.44 mmol, 1.0 equiv.) in EtOH (20 mL) was added to a solution of NH₄Cl (1.99 g, 37.2 mmol, 5.0 equiv.) and Fe (2.08 g, 37.2 mmol, 5.0 equiv.) in H₂O (10 mL) at RT. Then the solution was stirred at 80 °C for 1.5 hours under N₂. The reaction mixture was cooled to RT and filtered. The filtrate was concentrated under reduced pressure. Then residue was diluted with water (50 mL) and extracted with DCM (3 x 40 mL). The combined organic layers were washed with brine (70 mL), dried over Na₂SO₄, filtered, and concentrated under reduced pressure to afford the title compound (2.08 g, 91% yield) as a brown solid. LCMS [M+1] = 306.0. ¹H NMR (400 MHz, CHLOROFORM-*d*) δ 6.32 (dd, *J =* 2.2, 12.6 Hz, 1H), 4.14 (br s, 2H), 3.16 - 3.09 (m, 4H), 2.59 - 2.48 (m, 4H), 2.35 (s, 3H).

Step 3, 1-[6-amino-2,4-difluoro-3-(4-methylpiperazin-1-yl)phenyl]ethenone: To a solution of 2-bromo-3,5-difluoro-4-(4-methylpiperazin-1-yl)aniline (1 g, 3.27 mmol, 1.0 equiv.) in toluene (10 mL) was added tributyl(1-ethoxyvinyl)stannane (3.31 mL, 9.80 mmol, 3.0 equiv.) and Pd(PPh₃)₄ (377 mg, 327 µmol, 0.1 equiv.) under N₂. The mixture was stirred at 120 °C for 16 hours under N₂. The reaction mixture was cooled to RT and the reaction was quenched by the addition of an aqueous solution of KF (30 mL). The mixture was stirred at RT for 2 hours, diluted with water (10 mL) and extracted with EtOAc (3 x 20 mL). The combined organic layers were washed with brine (50 mL), dried over Na₂SO₄, filtered, and concentrated under reduced pressure. The residue was diluted in dioxane (15 mL) and 4N HCl in dioxane (2.08 mL, 1 equiv.) was added. The mixture was stirred at RT for 1 hour. The reaction mixture was quenched by the addition of saturated aqueous NaHCO₃ (50 mL), diluted with water (50 mL), and extracted with DCM (3 x 30 mL). The combined organic layers were washed with brine (60 mL), dried over Na₂SO₄, filtered, and concentrated under reduced pressure. The residue was purified by silica gel column chromatography (1:0 to 1:3 petroleum ether:ethyl acetate) to provide impure material which was repurified by preparative HPLC (column: Phenomenex luna C18 (250 x 70mm x 15 um); mobile phase: 15-35% acetonitrile in water (+ formic acid modifier)) to afford the title compound (190 mg, 8.5% yield) as a yellow solid. LCMS [M+1] = 270.2.

Step 4, N-[2-acetyl-3,5-difluoro-4-(4-methylpiperazin-1-yl)phenyl]-3-chloro-6-cyano-pyridine-2-carboxamide: A mixture of 3-chloro-6-cyano-pyridine-2-carboxylic acid (71 mg, 389 µmol, 1.0 equiv.) in SOCl₂ (2 mL) was purged with N₂ and then the mixture was stirred at 100 °C for 2 hours under N₂. The reaction mixture was concentrated under reduced pressure to provide 3-chloro-6-cyano-pyridine-2-carbonyl chloride (80 mg, crude) as a white solid which was used in the next phase of this step without further purification. A mixture of 3-chloro-6-cyano-pyridine-2-carbonyl chloride (77.6 mg, 386 µmol, 1.3 equiv.), 1-[6-amino-2,4-difluoro-3-(4-methylpiperazin-1-yl)phenyl]ethanone (80 mg, 297 µmol, 1.0 equiv.) in isopropyl acetate (3.5 mL) was purged with N₂, and then the mixture was stirred at 80 °C for 16 hours under N₂. The reaction mixture was concentrated under reduced pressure and the residue was purified by preparative TLC (10:1 DCM: MeOH) to afford the title compound (55 mg, 43% yield) as a yellow solid. LCMS [M+1] = 434.1. ¹H NMR (400 MHz, METHANOL-*d*₄) δ 8.26 (dd, *J* = 1.8, 14.2 Hz, 1H), 8.22 (d, *J* = 8.4 Hz, 1H), 8.02 (d, *J =* 8.4 Hz, 1H), 4.55 (br s, 4H), 3.38 - 3.34 (m, 4H), 2.69 (s, 3H), 2.63 (d, *J =* 7.6 Hz, 3H).

Step 5, 5-chloro-6-(5,7-difluoro-6-(4-methylpiperazin-1-yl)-4-oxo-1,4-dihydroquinolin-2-yl)picolinonitrile: To a solution of N-[2-acetyl-3,5-difluoro-4-(4-methylpiperazin-1-yl)phenyl]-3-chloro-6-cyano-pyridine-2-carboxamide (85 mg, 196 µmol, 1.0 equiv.) in 2-MeTHF (2 mL) was added LiOH (9.4 mg, 392 µmol, 2.0 equiv.). The reaction mixture was stirred at 90 °C for 12 hours. The reaction mixture was cooled to RT, and then the pH was adjusted to pH = 2-3 with aqueous 1M HCl. The aqueous phase was extracted with ethyl acetate (5 mL). The aqueous phase was purified by preparative HPLC (column: Phenomenex Luna C18 75 x 30mm x 3um; mobile phase: 1-40% acetonitrile in water (+formic acid modifier)) to afford the title compound (28.7 mg, 35% yield) as a yellow solid. LCMS [M+1] = 416.1. ¹H NMR (400 MHz, METHANOL-*d*₄) δ 8.32 (d, *J =* 8.4 Hz, 1H), 8.05 (d, *J* = 8.4 Hz, 1H), 7.28 (dd, *J =* 1.8, 11.8 Hz, 1H), 6.65 (s, 1H), 3.39 (br t, *J =* 4.6 Hz, 4H), 2.95 (br s, 4H), 2.62 (s, 3H).

The compounds in Table 4 were prepared following Scheme 1 using similar procedures to those described for Example 13.

**Table 4**

| Example number | Structure | IUPAC Name | ¹H NMR | LCMS Exact Mass |
|---|---|---|---|---|
| **14** | 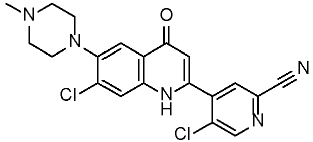 | 5-chloro-4-(7-chloro-6-(4-methylpiperazin-1-yl)-4-oxo-1,4-dihydroquinolin-2-yl)picolinonitrile | (400 MHz, DMSO-*d*₆ + D₂O) δ 9.02 (s, 1H), 8.37 (s, 1H), 7.74 (d, *J* = 6.8 Hz, 2H), 6.39 (br s, 1H), 3.06 (br s, 4H), 2.74 - 2.60 (m, 4H), 2.30 - 2.26 (m, 3H) | Calculated *m*/*z*=414.1, found *m*/*z* =414.0 |
| **15** | 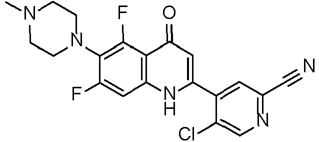 | 5-chloro-4-(5,7-difluoro-6-(4-methylpiperazin-1-yl)-4-oxo-1,4-dihydroquinolin-2-yl)picolinonitrile | (400 MHz, METHANOL-*d*₄) δ 8.93 (s, 1H), 8.18 (s, 1H), 7.16 (dd, *J =* 1.4, 11.8 Hz, 1H), 6.38 (s, 1H), 3.38 (br t, *J =* 4.4 Hz, 4H), 2.93 (br s, 4H), 2.60 (s, 3H) | Calculated *m*/*z*=416.1, found *m*/*z* =416.0 |
| **16** | 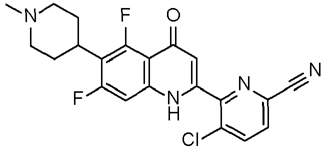 | 5-chloro-6-(5,7-difluoro-6-(1-methylpiperidin-4-yl)-4-oxo-1,4-dihydroquinolin-2-yl)picolinonitrile | (400 MHz, DMSO-d6) δ 9.93 (br s, 1H), 8.50 (d, J = 8.4 Hz, 1H), 8.30 (d, J = 8.4 Hz, 1H), 7.39 - 7.24 (m, 1H), 6.49 - 6.29 (m, 1H), 3.36 - 3.22 (m, 2H), 3.18 - 3.04 (m, 2H), 2.78 (d, J = 4.6 Hz, 3H), 2.69 - 2.63 (m, 1H), 2.32 - 2.22 (m, 2H), 1.95 (br d, J = 13.2 Hz, 2H) | Calculated *m*/*z*=415.1, found *m*/*z* =415.1 |
| **17** | 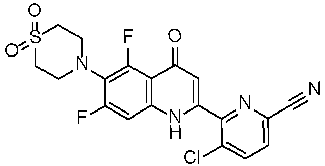 | 5-chloro-6-(6-(1,1-dioxidothiomorpholino)-5,7-difluoro-4-oxo-1,4-dihydroquinolin-2-yl)picolinonitrile | (400 MHz, DMSO-*d₆*) *δ* 11.9-12.2 (m, 1H), 8.49 (br d, *J =* 8.4 Hz, 1H), 8.28 (d, *J* = 8.4 Hz, 1H), 7.32 (br d, *J =* 7.6 Hz, 1H), 6.1-6.5 (m, 1H), 3.56 (br s, 4H), 3.26 (br d,*J* = 4.8 Hz, 4H) | Calculated *m*/*z*=451.0, found *m*/*z* =451.0 |
| **18** | 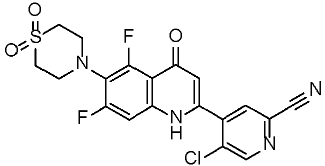 | 5-chloro-4-(6-(1,1-dioxidothiomorpholino)-5,7-difluoro-4-oxo-1,4-dihydroquinolin-2-yl)picolinonitrile | (400 MHz, DMSO-*d₆*) δ 12.15 (br s, 1H), 9.05 (s, 1H), 8.42 (s, 1H), 7.40 - 7.14 (m, 1H), 6.55 - 6.17 (m, 1H), 3.56 (br s, 4H), 3.37 - 3.20 (m, 4H) | Calculated *m*/*z*=451.0, found *m*/*z* =450.9 |
| **19** | 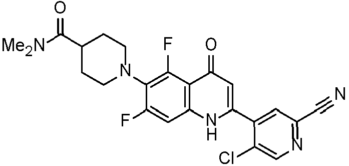 | 1-(2-(5-chloro-2-cyanopyridin-4-yl)-5,7-difluoro-4-oxo-1,4-dihydroquinolin-6-yl)-N,N-dimethylpiperidine-4-carboxamide | (400 MHz, DMSO-*d*₆) δ 9.06 (s, 1H), 8.43 (s, 1H), 7.24 (br d, *J=* 11.8 Hz, 1H), 6.51 - 6.13 (m, 1H), 3.19 (m, 4H), 3.06 (s, 3H), 2.83 (s, 3H), 2.81 - 2.74 (m, 1H), 1.76 - 1.64 (m, 4H) | Calculated *m*/*z*=472.1, found *m*/*z* =472.0 |
| **20** | 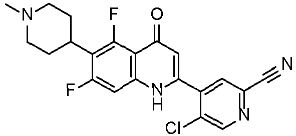 | 5-chloro-4-(5,7-difluoro-6-(1-methylpiperidin-4-yl)-4-oxo-1,4-dihydroquinolin-2-yl)picolinonitrile | (400 MHz, DMSO-*d*₆) δ 9.08 (s, 1H), 8.44 (s, 1H), 8.16 (s, 1H), 7.20 (br d, J = 11.8 Hz, 1H), 6.31 (br d, J = 2.5 Hz, 1H), 3.06 (br d, J = 9.9 Hz, 3H), 2.39 - 2.35 (m, 3H), 2.32 - 2.21 (m, 2H), 2.11 (q, J = 11.5 Hz, 2H), 1.74 (br d, J = 11.8 Hz, 2H) | Calculated *m*/*z*=415.1, found *m*/*z* =415.0 |
| **21** | 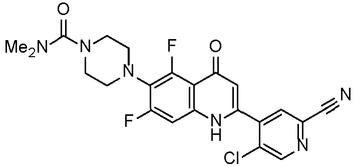 | 4-(2-(5-chloro-2-cyanopyridin-4-yl)-5,7-difluoro-4-oxo-1,4-dihydroquinolin-6-yl)-N,N-dimethylpiperazine-1-carboxamide | (400 MHz, METHANOL-*d₄*) *δ* 8.94 (s, 1H), 8.20 (s, 1H), 7.15 (br d, *J =* 11.4 Hz, 1H), 6.45 - 6.27 (m, 1H), 3.44 - 3.35 (m, 4H), 3.26 (br d, *J =* 3.8 Hz, 4H), 2.90 (s, 6H) | Calculated *m*/*z*=473.1, found *m*/*z* =473.1 |
| **22** | 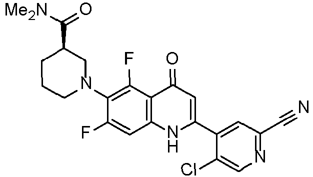 | (*R*)-1-(2-(5-chloro-2-cyanopyridin-4-yl)-5,7-difluoro-4-oxo-1,4-dihydroquinolin-6-yl)-N,N-dimethylpiperidine-3-carboxamide | (400 MHz, DMSO-*d*₆) δ 12.53 - 11.72 (m, 1H), 9.04 (s, 1H), 8.40 (s, 1H), 7.21 (br d, *J* = 12.0 Hz, 1H), 6.59 - 6.14 (m, 1H), 3.21 - 3.12 (m, 3H), 3.06 (s, 4H), 2.97 - 2.87 (m, 1H), 2.81 (s, 3H), 1.86 (br dd, *J =* 2.6, 12.6 Hz, 1H), 1.79 - 1.62 (m, 2H), 1.56 - 1.41 (m, 1H) | Calculated *m*/*z*=472.1, found *m*/*z* =472.0 |
| **23** | 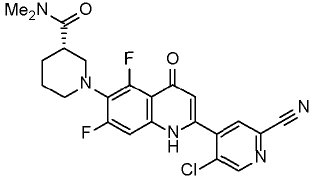 | (*S*)-1-(2-(5-chloro-2-cyanopyridin-4-yl)-5,7-difluoro-4-oxo-1,4-dihydroquinolin-6-yl)-N,N-dimethylpiperidine-3-carboxamide | (400 MHz, DMSO-*d*₆) δ 12.60 - 11.41 (m, 1H), 9.05 (s, 1H), 8.42 (s, 1H), 7.40 - 7.05 (m, 1H), 6.68 - 6.03 (m, 1H), 3.15 (br d, *J* = 5.6 Hz, 3H), 3.05 (s, 4H), 2.93 - 2.86 (m, 1H), 2.81 (s, 3H), 1.85 (br d, *J =* 10.4 Hz, 1H), 1.77 - 1.61 (m, 2H), 1.48 (br dd, *J =* 4.6, 12.2 Hz, 1H) | Calculated m/z=472.1, found *m*/*z* =472.0 |
| **24** | 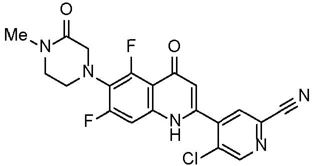 | 5-chloro-4-(5,7-difluoro-6-(4-methyl-3-oxopiperazin-1-yl)-4-oxo-1,4-dihydroquinolin-2-yl)picolinonitrile | (400 MHz, METHANOL-*d*₄) δ 8.94 (s, 1H), 8.25 - 8.15 (m, 1H), 7.18 (br d, *J =* 11.6 Hz, 1H), 6.45 - 6.28 (m, 1H), 3.95 - 3.83 (m, 2H), 3.58 - 3.45 (m, 3H), 3.46 - 3.44 (m, 1H), 3.04 (s, 3H) | Calculated *m*/*z*=430.1, found *m*/*z* =430.0 |
| **25** | 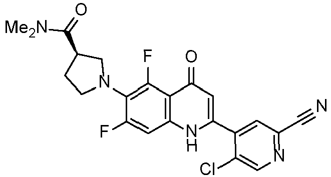 | (*R*)-1-(2-(5-chloro-2-cyanopyridin-4-yl)-5,7-difluoro-4-oxo-1,4-dihydroquinolin-6-yl)-N,N-dimethylpyrrolidine-3-carboxamide | (400 MHz, DMSO-*d*₆) δ 12.21 - 11.51 (m, 1H), 9.03 (s, 1H), 8.41 (s, 1H), 7.54 - 6.96 (m, 1H), 6.75 - 5.68 (m, 1H), 3.60 (br s, 3H), 3.54 - 3.39 (m, 2H), 3.06 (s, 3H), 2.86 (s, 3H), 2.18 - 2.08 (m, 1H), 2.07 - 1.97 (m, 1H) | Calculated *m*/*z*=458.1, found *m*/*z* =458.1 |
| **26** | | (*S*)-1-(2-(5-chloro-2-cyanopyridin-4-yl)-5,7-difluoro-4-oxo-1,4-dihydroquinolin-6-yl)-N,N-dimethylpyrrolidine-3-carboxamide | (400 MHz, DMSO-*d*₆) δ 11.90 (br s, 1H), 9.04 (s, 1H), 8.42 (br s, 1H), 7.38 - 7.04 (m, 1H), 6.67 - 5.79 (m, 1H), 3.61 (br s, 3H), 3.49 - | Calculated *m*/*z*=458.1, found *m*/*z* =458.1 |
| | 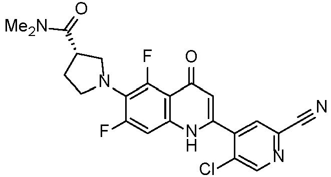 | | 3.39 (m, 2H), 3.06 (s, 3H), 2.86 (s, 3H), 2.18 - 2.08 (m, 1H), 2.07 - 1.98 (m, 1H) | |

### Example 27

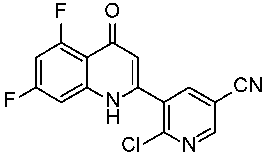

### 6-chloro-5-(5,7-difluoro-4-oxo-1,4-dihydroquinolin-2-yl)nicotinonitrile

Step 1, 2-hydroxy-5-iodonicotinic acid: To a solution of methyl 2-chloro-5-iodonicotinate (3.0 g, 10.7 mmol, 1.0 equiv.) in THF (20 mL) and H₂O (7 mL) was added LiOH·H₂O (1.3 g, 32.2 mmol, 3.0 equiv.). The mixture was stirred at 50 °C for 16 hours. The mixture was concentrated under reduced pressure to remove THF. The pH of the mixture was adjusted to pH = 6 with aqueous 1 M HCl. During the pH adjustment a white solid precipitated and was collected by filtration. The filter cake was washed with H₂O (10 mL) and concentrated in vacuum to give 2-hydroxy-5-iodonicotinic acid (2.5 g, 91% yield) as a white solid. LCMS [M+1] = 265.9. ¹H NMR (400 MHz, DMSO-*d₆*) δ 8.28 (d, *J =* 2.4 Hz, 1H), 8.21 (d, *J* = 2.4 Hz, 1H).

Step 3, N-(2-acetyl-3,5-difluorophenyl)-2-chloro-5-iodonicotinamide: A solution of 2-hydroxy-5-iodonicotinic acid (2.5 g, 9.7 mmol, 1.0 equiv.) in POCl₃ (15 mL) was stirred at 90 °C for 5 hours. The reaction mixture was concentrated under reduced pressure to remove volatiles and afford the title compound (3 g, crude) as a white solid which was used in the next phase of the reaction without further purification.

To a solution of 1-(2-amino-4,6-difluorophenyl)ethanone (1.4 g, 8.7 mmol, 1.0 equiv.) in THF (15 mL) was added NaH (384.2 mg, 9.6 mmol, 1.1 equiv.; 60% dispersion in oil). Then 2-chloro-5-iodonicotinoyl chloride (2.9 g, 9.61 mmol, 1.1 equiv.) was added to the mixture. The mixture was stirred at RT for 16 hours. The reaction mixture was quenched by the addition of a saturated aqueous solution of NH₄Cl (10 mL). The aqueous layer was extracted with ethyl acetate (2 x 40 mL) and the combined organic layers were washed with brine (15 mL), dried over anhydrous Na₂SO₄, filtered, and concentrated under reduced pressure. The residue was triturated with ethyl acetate at 25 °C for 0.5 hours to afford the title compound (3.0 g, 79% yield) as a white solid. LCMS [M+1] = 436.9. ¹H NMR (400 MHz, DMSO-*d₆*) δ 11.34 (s, 1H), 8.80 (d, *J* = 2.4 Hz, 1H), 8.36 (d, *J* = 2.4 Hz, 1H), 7.50 - 7.40 (m, 1H), 7.30 (ddd, *J* = 2.6, 8.8, 11.2 Hz, 1H), 2.54 (d, *J=* 3.8 Hz, 3H).

Step 4, 2-(2-chloro-5-iodopyridin-3-yl)-5,7-difluoroquinolin-4(1H)-one: To a solution of N-(2-acetyl-3,5-difluorophenyl)-2-chloro-5-iodonicotinamide (740 mg, 1.6 mmol, 1.0 equiv.) in dioxane (7 mL) was added LiOH (40.6 mg, 1.6 mmol, 1.0 equiv.). The mixture was stirred at 110 °C for 16 hours. The reaction mixture was cooled to RT and diluted with H₂O (5 mL) and extracted with ethyl acetate (3 x 10 mL). The combined organic layers were washed with brine (5 mL), dried over anhydrous Na₂SO₄, filtered, and concentrated under reduced pressure. The residue was triturated with acetonitrile at RT for 0.5 hours to afford the title compound (200 mg, 28% yield) as a white solid. LCMS [M+1] = 418.9. ¹H NMR (400 MHz, DMSO-*d₆*) δ 12.11 (br s, 1H), 8.85 (d, *J=* 2.0 Hz, 1H), 8.52 (d, *J=* 1.6 Hz, 1H), 7.12 (br d, *J* = 8.2 Hz, 2H), 6.10 (br s, 1H).

Step 5, 6-chloro-5-(5,7-difluoro-4-oxo-1,4-dihydroquinolin-2-yl)nicotinonitrile: To a solution of 2-(2-chloro-5-iodopyridin-3-yl)-5,7-difluoroquinolin-4(1H)-one (150 mg, 358 µmol, 1.0 equiv.) in DMA (3 mL) was added Zn(CN)₂ (25.2 mg, 215 µmol, 0.6 equiv.) and Pd(PPh₃)₄ (41.4 mg, 35.8 µmol, 0.1 equiv.) under N₂. The mixture was stirred at 120 °C for 3 hours under N₂. The reaction mixture was cooled to RT and then poured into water (5 mL). The aqueous phase was extracted with ethyl acetate (2 x 10 mL). The combined organic layers were washed with brine (2 x 10 mL), dried with anhydrous Na₂SO₄, filtered, and concentrated under reduced pressure. The residue was purified by preparative HPLC (column: Phenomenex C18 75 x 30mm x 3um; mobile phase: 10-40% acetonitrile in water (+ NH₄HCO₃ modifier)) to afford the title compound (52.1 mg, 44% yield) as a white solid. LCMS [M+1] = 317.9. ¹H NMR (400 MHz, DMSO-*d₆*) δ 12.18 (br d, *J =* 2.2 Hz, 1H), 9.11 (d, *J =* 2.2 Hz, 1H), 8.73 (d, *J =* 2.2 Hz, 1H), 7.16 (br d, *J =* 10.6 Hz, 2H), 6.35 - 5.92 (m, 1H).

### Example 28

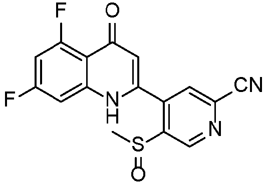

4-(5,7-difluoro-4-oxo-1,4-dihydroquinolin-2-yl)-5-(methylsulfinyl)picolinonitrile 4-(5,7-difluoro-4-oxo-1,4-dihydroquinolin-2-yl)-5-(methylsulfinyl)picolinonitrile: To a mixture of 4-(5,7-difluoro-4-oxo-1H-quinolin-2-yl)-5-methylsulfanyl-pyridine-2-carbonitrile (60 mg, 182 µmol, 1 equiv.) in DCM (1 mL) was added *m*-CPBA (55.4 mg, 273 µmol, 85% purity, 1.5 equiv.) at RT under N₂. The mixture was stirred at RT for 16 hours. The reaction mixture was poured into a saturated aqueous solution of Na₂SO₃ (15 mL). The aqueous phase was extracted with ethyl acetate (3 x 20 mL). The combined organic layers were washed with brine (2 x 20 mL), dried with anhydrous Na₂SO₄, filtered, and concentrated under reduced pressure. The crude product was purified by preparative HPLC two times (1-column: Waters Xbridge BEH C18 100 x 30mm x 10um; mobile phase: 10-35% acetonitrile in water (+NH₄HCO₃ modifier)-ACN]; and 2- column: Phenomenex Luna C18 150 x 30mm x 5um; mobile phase: 1-35% acetonitrile in water (+ formic acid modifier) to afford the title compound (8.4 mg, 13% yield) as off-white solid. LCMS [M+1] = 346.0. ¹H NMR (400 MHz, DMSO-*d*₆) δ 9.37 (s, 1H), 8.60 (s, 1H), 7.73 - 7.51 (m, 1H), 7.48 - 7.20 (m, 2H), 2.52 (br d, *J* = 1.8 Hz, 3H).

### Example 29

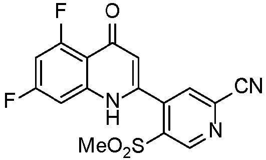

4-(5,7-difluoro-4-oxo-1,4-dihydroquinolin-2-yl)-5-(methylsulfonyl)picolinonitrile 4-(5,7-difluoro-4-oxo-1,4-dihydroquinolin-2-yl)-5-(methylsulfonyl)picolinonitrile: To a mixture of 4-(5,7-difluoro-4-oxo-1H-quinolin-2-yl)-5-methylsulfanyl-pyridine-2-carbonitrile (110 mg, 334 µmol, 1.0 equiv.) in acetone (1 mL), water (0.6 mL), methanol (0.7 mL) and THF (0.7 mL) was added Oxone (616.03 mg, 1.00 mmol, 3.0 equiv.). The mixture was stirred at 40 °C for 32 hours. The reaction mixture was poured into H₂O (15 mL). The aqueous phase was extracted with ethyl acetate (3 x 20 mL). The combined organic layers were washed with brine (2 x 20 mL), dried with anhydrous Na₂SO₄, filtered, and concentrated. The residue was purified by preparative HPLC (column: Phenomenex Luna C18 75 x 30mm x 3um; mobile phase: 20-50% acetonitrile in water (+formic acid modifier) to afford the title compound (16.8 mg, 14% yield) as an off-white solid. LCMS [M+1] = 361.9. ¹H NMR (400 MHz, DMSO-*d*₆) δ 11.96 (br s, 1H), 9.35 (br s, 1H), 8.43 (br s, 1H), 7.27 - 6.88 (m, 2H), 6.18 (br s, 1H), 3.75 - 3.38 (m, 3H).

### Example 30

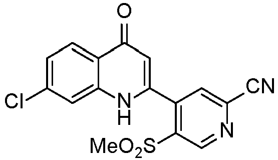

### 4-(7-chloro-4-oxo-1,4-dihydroquinolin-2-yl)-5-(methylsulfonyl)picolinonitrile

Step 1, N-(2-acetyl-5-chloro-phenyl)-2-cyano-5-methylsulfanyl-pyridine-4-carboxamide: To a solution of 1-(2-amino-4-chloro-phenyl)ethanone (262 mg, 1.5 mmol, 1.0 equiv.) in isopropyl acetate (3 mL) was added 2-cyano-5-methylsulfanyl-pyridine-4-carbonyl chloride (328.5 mg, 1.54 mmol, 1.0 equiv.). The mixture was stirred at 80 °C for 16 hours. The reaction mixture was cooled to RT and the volatiles were removed under reduced pressure. The residue was triturated with MTBE at RT for 30 minutes. The mixture was filtered and the filter cake was concentrated under reduced pressure to afford the title compound (440 mg, 82% yield) as a white solid. LCMS [M+1] = 346.0. ¹HNMR (400 MHz, DMSO-*d*₆) δ 11.90 (s, 1H), 8.82 (s, 1H), 8.38 (d, *J* = 2.2 Hz, 1H), 8.23 (s, 1H), 8.08 (d, *J* = 8.6 Hz, 1H), 7.44 (dd, *J =* 2.2, 8.6 Hz, 1H), 2.65 (d, *J* = 7.8 Hz, 6H).

Step 2, 4-(7-chloro-4-oxo-1H-quinolin-2-yl)-5-methylsulfanyl-pyridine-2-carbonitrile: To a solution of N-(2-acetyl-5-chloro-phenyl)-2-cyano-5-methylsulfanyl-pyridine-4-carboxamide (345 mg, 998 µmol, 1.0 equiv.) in dioxane (3 mL) was added LiOH (23.8 mg, 998 µmol, 1.0 equiv.). The mixture was stirred at 110 °C for 12 hours. The pH of the mixture was adjusted pH = 3-4 with aqueous 1M HCl. Then the mixture was diluted with water (3mL) and a precipitate formed. The mixture was filtered and concentrated under reduced pressure to give a residue. The crude product was triturated with acetonitrile at RT for 30 minutes to afford the title compound (235 mg, 72% yield) as a white solid. LCMS [M+1] = 328.1. ¹H NMR (400 MHz, DMSO-*d*₆) δ 12.08 (br s, 1H), 8.81 (s, 1H), 8.21 (s, 1H), 8.12 (d, *J* = 8.6 Hz, 1H), 7.58 (d, *J =* 1.6 Hz, 1H), 7.42 (dd, *J* = 1.8, 8.6 Hz, 1H), 6.17 (s, 1H), 2.74 - 2.60 (m, 3H).

Step 3, 4-(7-chloro-4-oxo-1,4-dihydroquinolin-2-yl)-5-(methylsulfonyl)-picolinonitrile: To a solution of 4-(7-chloro-4-oxo-1H-quinolin-2-yl)-5-methylsulfanyl-pyridine-2-carbonitrile (288 mg, 879 µmol, 1.0 equiv.) in acetone (0.35 mL), H₂O (0.2 mL), MeOH (0.25 mL), THF (0.25 mL) was added Oxone (1.6 g, 2.6 mmol, 3.0 equiv.) The mixture was stirred at 40 °C for 16 hours. The mixture was quench by addition of saturated aqueous Na₂SO₃ (111 mg, 879 µmol, 1.0 equiv.). Then the mixture was diluted with water (5 mL) and filtered. The filtrate was concentrated in vacuum and the residue was purified by preparative HPLC (column: Phenomenex Luna C18 200 x 40mm x 10um; mobile phase: 20-50% acetonitrile in water (+formic acid modifier)) to afford the title compound (74.7 mg, 24% yield) as a white solid. LCMS [M+1] = 360.8. ¹H NMR (400 MHz, DMSO-*d*₆) δ 12.77 - 11.60 (m, 1H), 9.35 (s, 1H), 8.54 (br s, 1H), 8.18 (br d, *J=* 8.8 Hz, 1H), 8.00 - 7.29 (m, 2H), 7.08 - 5.99 (m, 1H), 3.54 (br s, 3H).

The compounds in Table 5 were prepared following Scheme 1 using similar procedures to those described for Example 30.

**Table 5**

| Example number | Structure | IUPAC Name | ¹H NMR | LCMS Exact Mass |
|---|---|---|---|---|
| **31** | 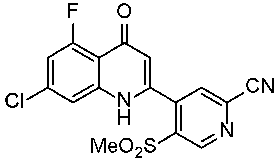 | 4-(7-chloro-5-fluoro-4-oxo-1,4-dihydroquinolin-2-yl)-5-(methylsulfonyl)picolinonitrile | (400 MHz, DMSO-*d₆*) δ 9.35 (s, 1H), 8.54 (s, 1H), 7.74 - 7.51 (m, 1H), 7.49 - 7.29 (m, 1H), 6.88 - 6.35 (m, 1H), 3.55 (br s, 3H) | Calculated m/z=378.0, found *m*/*z* =378.0 |
| **32** | 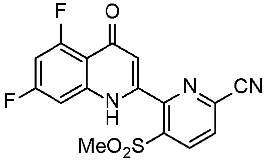 | 6-(5,7-difluoro-4-oxo-1,4-dihydroquinolin-2-yl)-5-(methylsulfonyl)picolinonitrile | (400 MHz, DMSO-*d₆*) δ 8.77 (d, *J* = 8.4 Hz, 1H), 8.50 (d, *J =* 8.4 Hz, 1H), 7.48 - 7.22 (m, 2H), 6.82 (br s, 1H), 3.62 (s, 3H) | Calculated *m*/*z=362.0,* found *m*/*z* =361.9 |

### Example 33

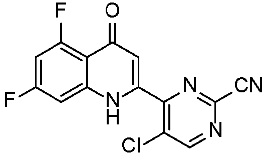

### 5-chloro-4-(5,7-difluoro-4-oxo-1,4-dihydroquinolin-2-yl)pyrimidine-2-carbonitrile

Step 1, N-(2-acetyl-3,5-difluoro-phenyl)-5-chloro-2-methylsulfanyl-pyrimidine-4-carboxamide: A mixture of 5-chloro-2-methylsulfanyl-pyrimidine-4-carboxylic acid (1.4 g, 6.84 mmol, 1.0 equiv.) in SOCl₂ (20 mL) was purged with N₂ and then the mixture was stirred at 80 °C for 0.5 hours under N₂. The reaction mixture was cooled to RT and concentrated under reduced pressure to give 5-chloro-2-methylsulfanyl-pyrimidine-4-carbonyl chloride (1.45 g, crude) as a black solid. The product was used directly in the next phase of the reaction without further purification.

To a solution of 1-(2-amino-4,6-difluoro-phenyl)ethanone (1.0 g, 5.8 mmol, 1.0 equiv.) in isopropyl acetate (20 mL) was added 5-chloro-2-methylsulfanyl-pyrimidine-4-carbonyl chloride (1.4 g, 6.4 mmol, 1.1 equiv.). The mixture purged with N₂ and then was stirred at 80 °C for 2 hours under N₂. The mixture was cooled to RT and then diluted with H₂O (5 mL) and filtered and concentrated under reduced pressure. The residue was triturated with MTBE at RT for 5 minutes to afford the title compound (1.9 g, 90% yield) as a white solid. LCMS [M+1] = 358.0. ¹H NMR (400 MHz, CHLOROFORM-*d*) *δ* 13.16 (br s, 1H), 8.69 (s, 1H), 8.61 - 8.52 (m, 1H), 6.70 (ddd, *J =* 2.6, 8.2, 11.8 Hz, 1H), 2.80 (s, 3H), 2.68 (d, *J* = 8.0 Hz, 3H).

Step 2, 2-(5-chloro-2-methylsulfanyl-pyrimidin-4-yl)-5,7-difluoro-1H-quinolin-4-one: To a solution of N-(2-acetyl-3,5-difluoro-phenyl)-5-chloro-2-methylsulfanyl-pyrimidine-4-carboxamide (1.0 g, 2.8 mmol, 1.0 equiv.) in dioxane (10 mL) was added LiOH (134 mg, 5.6 mmol, 2.0 equiv.). The mixture was purged with N₂ and then the mixture was stirred at 110 °C for 6 hours under N₂. The pH of the reaction mixture was adjusted to pH = 6 with aqueous 1M HCl and diluted with H₂O (5 mL). The precipitate was filtered off and the filter cake was triturated with MTBE at RT for 5 minutes to afford the title compound (720 mg, 76% yield) as a white solid. LCMS [M+1] = 340.0. ¹H NMR (400 MHz, METHANOL-*d4*) *δ* 8.84 (s, 1H), 7.26 (br d, *J =* 9.4 Hz, 1H), 7.05 - 6.95 (m, 1H), 6.73 (br s, 1H), 2.63 (s, 3H).

Step 3, 2-(5-chloro-2-methylsulfonyl-pyrimidin-4-yl)-5,7-difluoro-1H-quinolin-4-one: To a solution of 2-(5-chloro-2-methylsulfanyl-pyrimidin-4-yl)-5,7-difluoro-1H-quinolin-4-one (300 mg, 883 µmol, 1.0 equiv.) in acetone (15 mL), H₂O (8 mL), MeOH (1 mL) and THF (1 mL) was added Oxone (1.6 g, 2.6 mmol, 3.0 equiv.). The mixture was purged with N₂ and then stirred at RT for 2 hours under N₂. The reaction mixture was diluted with H₂O (20 mL) and the aqueous layer was extracted with ethyl acetate (5 x 15 mL). The combined organic layers were washed with brine (20 mL), dried over Na₂SO₄, filtered, and concentrated under reduced pressure. The residue was purified by silica gel column chromatography (3:1 to 0:1 petroleum ether:ethyl acetate) to afford the title compound (185 mg, 52% yield) as a yellow solid. LCMS [M+1] = 371.9. ¹H NMR (400 MHz, METHANOL-*d₄*) δ 9.31 (s, 1H), 7.29 (br d, *J =* 7.8 Hz, 1H), 7.03 (br t, *J* = 10.0 Hz, 1H), 6.90 (br s, 1H), 3.47 (s, 3H).

Step 4, 5-chloro-4-(5,7-difluoro-4-oxo-1,4-dihydroquinolin-2-yl)pyrimidine-2-carbonitrile: To a solution of 2-(5-chloro-2-(methylsulfonyl)pyrimidin-4-yl)-5,7-difluoroquinolin-4(1H)-one (50.0 mg, 135 µmol, 1.0 equiv.) in DMSO (2.5 mL) cooled to 10 °C was added NaCN (16.5 mg, 336 µmol, 2.5 equiv.) and the mixture was stirred at 10 °C for 10 minutes. The reaction was diluted with H₂O (10 mL) and the aqueous phase was extracted with ethyl acetate (10 x 15 mL). The combined organic phase layers were washed with brine (2 x 10 mL), dried with anhydrous Na₂SO₄, filtered, and concentrated. The residue was purified by preparative HPLC (column: Phenomenex Luna C18 75 x 30mm x 3um; mobile phase: 10-50% acetonitrile in water (+formic acid modifier)) to afford the title compound (18.2 mg, 42% yield) as an off-white solid. LCMS [M+1] = 318.9. ¹H NMR (400 MHz, DMSO-*d*₆) δ 12.58 - 11.84 (m, 1H), 9.42 (s, 1H), 7.43 (br d, *J =* 9.8 Hz, 1H), 7.27 (br s, 1H), 6.96 - 6.49 (m, 1H).

### Example 34

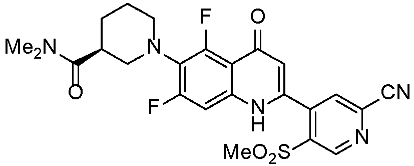

### (S)-1-(2-(2-cyano-5-(methylsulfonyl)pyridin-4-yl)-5,7-difluoro-4-oxo-1,4-dihydroquinolin-6-yl)-N,N-dimethylpiperidine-3-carboxamide

Step 1, N-[2-acetyl-4-[3-(dimethylcarbamoyl)-1-piperidyl]-3,5-difluoro-phenyl]-5-chloro-2-cyano-pyridine-4-carboxamide: To a solution of 1-(3-acetyl-4-amino-2,6-difluorophenyl)-N,N-dimethyl-piperidine -3-carboxamide (300 mg, 922 µmol, 1.0 equiv.) in isopropyl acetate (5 mL) was added 5-chloro-2-cyano-pyridine-4-carbonyl chloride (222 mg, 1.1 mmol, 1.2 equiv.) at RT under N₂. The mixture was stirred at 80 °C for 2 hours. The reaction mixture cooled to RT and was poured into water (20 mL). The aqueous phase was extracted with ethyl acetate (3 x 20 mL). The combined organic layers were washed with brine, dried with anhydrous Na₂SO₄, filtered, and concentrated under reduced pressure. The residue was purified by silica gel column chromatography (50:1 to 1:1 petroleum ether:ethyl acetate) to afford the title compound (360 mg, 80% yield) as yellow solid. LCMS [M+1] = 490.2. ¹H NMR (400 MHz, CHLOROFORM-*d*) δ 12.13 (s, 1H), 8.82 (s, 1H), 8.39 (dd, *J =* 1.8, 13.6 Hz, 1H), 7.90 (s, 1H), 3.41 - 3.31 (m, 1H), 3.24 - 3.14 (m, 3H), 3.14 - 3.10 (m, 3H), 2.96 (s, 4H), 2.71 - 2.63 (m, 3H), 2.00 - 1.88 (m, 1H), 1.87 - 1.66 (m, 3H).

Step 2, 1-[2-(5-chloro-2-cyano-4-pyridyl)-5,7-difluoro-4-oxo-1H-quinolin-6-yl]-N,N-dimethyl-piperidine-3-carboxamide: To a mixture of N-[2-acetyl-4-[3-(dimethylcarbamoyl)-1-piperidyl]-3,5-difluoro-phenyl] -5-chloro-2-cyano-pyridine-4-carboxamide (360 mg, 735 µmol, 1.0 equiv.) in 2-MeTHF (5 mL) was added LiOH (52.8 mg, 2.2 mmol, 3.0 equiv.) at RT under N₂. The mixture was stirred at 80 °C for 16 hours. The pH of the reaction mixture was adjusted to pH = 5-6 with aqueous 1N HCl. The mixture was poured into ice-water (70 mL) and the aqueous phase was extracted with ethyl acetate (2 x 70 mL). The combined organic layers were washed with brine (10 mL), dried with anhydrous Na₂SO₄, filtered, and concentrated under reduced pressure. The residue was purified by silica gel column chromatography (100:1 to 30:1 DCM:MeOH) to afford the title compound (185 mg, 53% yield) as yellow solid. LCMS [M+1] = 472.2. ¹H NMR (400 MHz, DMSO-*d*₆) δ 12.13 - 11.89 (m, 1H), 9.15 - 8.94 (m, 1H), 8.46 (s, 1H), 7.21 - 7.06 (m, 1H), 6.08 (s, 1H), 3.11 (br d, *J =* 6.4 Hz, 3H), 3.07 - 2.97 (m, 4H), 2.97 - 2.84 (m, 1H), 2.80 (s, 3H), 1.85 (br d, *J= 11.8* Hz, 1H), 1.78 - 1.62 (m, 2H), 1.58 - 1.39 (m, 1H).

Step 3, (S)-1-(2-(2-cyano-5-(methylthio)pyridin-4-yl)-5,7-difluoro-4-oxo-1,4-dihydroquinolin-6-yl)-N,N-dimethylpiperidine-3-carboxamide: To a mixture of 1-(2-(5-chloro-2-cyanopyridin-4-yl)-5,7-difluoro-4-oxo-1,4- dihydroquinolin-6-yl)-N,N-dimethylpiperidine-3-carboxamide (182 mg, 386 µmol, 1.0 equiv.) in DMF (3 mL) was added NaSMe (67.6 mg, 964 µmol, 2.5 equiv.) in one portion at 0 °C under N₂. The mixture was stirred at 0 °C for 1 hour. The reaction mixture was poured into ice water (20 mL) and stirred for 10 minutes. The aqueous phase was extracted with ethyl acetate (2 x 20 mL). The combined organic layers were washed with brine (3 mL), dried with anhydrous Na₂SO₄, filtered, and concentrated under reduced pressure. Additional material was extracted from the aqueous phase by adjusting the pH to 5-6 with aqueous 1M HCl and collecting the precipitate that formed. The combined crude product was triturated with acetonitrile. The crude racemic title compound was resolved into the enantiomers by SFC (column: DAICEL CHIRALCEL OJ (250mm x 30mm x 10um); mobile phase: 25% MeOH in CO₂ (+0.1%NH₃H₂O) to afford (R)-1-(2-(2-cyano-5-(methylthio)pyridin-4-yl) -5,7-difluoro-4-oxo-1,4-dihydroquinolin-6-yl)-N,N-dimethylpiperidine-3-carboxamide (early-eluting enantiomer) (35.0 mg, 19% yield) and the title compound (late-eluting enantiomer) (35.0 mg, 19% yield) as yellow solids. LCMS [M+1] = 484.0. ¹H NMR (400 MHz, DMSO-*d*₆) δ 12.52 - 11.50 (m, 1H), 8.78 (s, 1H), 8.14 (s, 1H), 7.48 - 7.01 (m, 1H), 6.46 - 5.96 (m, 1H), 3.14 (br d, *J =* 6.2 Hz, 3H), 3.05 (s, 4H), 2.95 - 2.85 (m, 2H), 2.81 (s, 3H), 2.65 (s, 3H), 1.85 (br d, *J* = 11.0 Hz, 1H), 1.79 - 1.61 (m, 2H), 1.57 - 1.40 (m, 1H).

Step 4, (*S*)-1-(2-(2-cyano-5-(methylsulfonyl)pyridin-4-yl)-5,7-difluoro-4-oxo-1,4-dihydroquinolin-6-yl)-N,N-dimethylpiperidine-3-carboxamide: To a solution of (*S*)-1-(2-(2-cyano-5-(methylthio)pyridin-4-yl)-5,7-difluoro-4-oxo-1,4-dihydroquinolin-6-yl)-N,N-dimethylpiperidine-3-carboxamide (31.1 mg, 64.2 µmol, 1.0 equiv.) in DCM (0.5 mL) cooled to 0 °C was added *m*-CPBA (39.1 mg, 193 µmol, 3.0 equiv.; 85% purity). The mixture was stirred at RT for 3 hours. The reaction was quenched by the addition of a saturated aqueous solution of Na₂SO₃. The mixture was concentrated in vacuum. The residue was diluted with DMF (2 mL) and filtered to remove the solids. The filtrate was purified by preparative HPLC (column: Phenomenex Luna C18 75 x 30mm x 3um; mobile phase: 30-60% acetonitrile in water (+formic acid modifier) to afford the title compound (4.0 mg, 7.5 µmol, 12% yield) as yellow solid. LCMS [M+1] = 516.1. ¹H NMR (400 MHz, METHANOL-*d*₄) δ 9.40 (s, 1H), 8.29 (br s, 1H), 7.26 - 6.82 (m, 1H), 6.47 - 6.12 (m, 1H), 3.28-3.05 (m, 10H), 3.14 - 3.05 (m, 1H), 3.01 (s, 3H), 1.98 (br dd, *J =* 3.2, 12.8 Hz, 1H), 1.85 (br s, 2H), 1.74 - 1.58 (m, 1H).

### Biochemical and Cellular Assays

### PPARγ-NCOR1 recruitment assay:

Compound potency (EC₅₀) and maximal extent of NCOR1 recruitment to PPARG were assessed a TR-FRET binding assay measuring association of a biotinylated NCOR1 ID2 peptide (Biotin-GHSFADPASNLGLEDIIRK ALMG-amide) to PPARG/RXRA LBD heterodimer. Specifically, a 20 microliters of TR-FRET master mix consisting of 2 nM WT PPARG LBD (e. coli expressed, His-TEV-Q203-Y477; Uniprot ID P37231-2), 2 nM WT RXRA LBD or mutant S427F RXRA LBD (e. coli expressed, Flag-TEV-E228-T462; P19793-1), 50 nM NCOR1, 80 nM Rosiglitazone, 25 nM streptavidin-d2 (Cisbio) and 0.3 nM Anti-His Tb (Cisbio) in 25 mM MOPS pH 7.4, 25 mM KCl, 1 mM EDTA, 0.01% BSA, 0.01% Tween-20 and 1 mM TCEP was added to 384-well plates containing duplicate 10-point dose response titrations of compounds in 60 nL DMSO (0.3% f.c. DMSO (v/v)). Mixtures were incubated for 3 hours and read in an EnVision plate reader (Perkin Elmer) with Ex/Em 615/665. To determine the potency (EC₅₀) and extent of NCOR1 recruitment, TR-FRET ratios were normalized to the average ratio of DMSO control wells (0%) and to the average maximum ratio for positive control compound (T0070907 (2-chloro-5-nitro-N-4-pyridinyl-benzamide); defined as 100%) in CDD Vault and analyzed using the Levenberg-Marquardt algorithm.

### PPARγ-MED1 blockade assay:

Compound potency (IC₅₀) and maximal extent of MED1 repulsion to PPARG were assessed a TR-FRET binding assay measuring association of a biotinylated MED1 LxxLL peptide (Biotin- VSSMAGNTKNHPMLMNLLKDNPAQ-amide) to PPARG/RXRA LBD heterodimer. Specifically, a 20 microliters of TR-FRET master mix consisting of 2 nM WT PPARG LBD (e. coli expressed, His-TEV-Q203-Y477; Uniprot ID P37231-2), 2 nM WT RXRA LBD (e. coli expressed, Flag-TEV-E228-T462; P19793-1), 350 nM NCOR1, 80 nM Rosiglitazone, 175 nM streptavidin-d2 (Cisbio) and 0.3 nM Anti-His Tb (Cisbio) in 25 mM MOPS pH 7.4, 25 mMKCl, 1 mM EDTA, 0.01% BSA, 0.01% Tween-20 and 1 mM TCEP was added to 384-well plates containing duplicate 10-point dose response titrations of compounds in 60 nL DMSO (0.3% DMSO f.c. (v/v)). Mixtures were incubated for 3 hours and read in an EnVision plate reader (Perkin Elmer) with Ex/Em 615/665. To determine the potency (IC₅₀) and extent of MED1 repulsion, TR-FRET ratios were normalized to the average ratio of DMSO control wells (0%) and to the average minimum ratio for positive control compound (GW9662 (2-chloro-5-nitrobenzanilide); defined as 100%) in CDD Vault and analyzed using the Levenberg-Marquardt algorithm.

### Bladder Cancer Pharmacodynamic Assay

5637 (PPARG amplified) and HT1197 (RXRA S427F mutation) cells were used for assessment of modulation of PPARG target genes using quantitative PCR. Cells were treated for 24 hours with PPARG inverse agonists prior to analysis of FABP4 (IDT, Cat: Hs.PT 58.20106818) and ANGPTL4 (IDT, Cat: Hs.PT 58.25480012) expression, with expression of the housekeeping gene TBP (IDT, Cat: Hs.PT 58v.39858774) used to normalize expression across samples. Quantitative PCR was performed using an ABI QuantStudio 7 Flex Reaction system. Data were analyzed and reported relative to DMSO control using the comparative Ct method (ΔΔCt).

### Table 6

For the PPARG-NCOR recruitment assay the EC₅₀ is expressed as follows, A: <10 nM, B: 10-100 nM, C: 100-1,000 nM, D: 1,000-10,000 nM, E: >10,000 nM. The % NCOR recruitment is expressed as follows, A: >100% (> the control compound, T907), B: <100% (< the control compound, T907).

For the PPARG-MED1 recruitment assay the EC₅₀ is expressed as follows, A: <10 nM, B: 10-100 nM, C: 100-1,000 nM, D: 1,000-10,000 nM, E: >10,000 nM. The % MED1 blockade is expressed as follows, A: >100% (> the control compound, GW9662), B: <100% (< the control compound, GW9662).

For the HT1197 cell assay the EC₅₀ is expressed as follows, A: <10 nM, B: 10-100 nM, C: 100-1,000 nM, D: 1,000-10,000 nM, E: >10,000 nM, ND: not determined. The % inhibition of ANGPTL4, a PPARG target gene, at 100 nM compound concentration is expressed as percentage of a DMSO control experiment.

| *Example number* | *PPARG-NCOR recruitment assay EC₅₀ - %NCOR recruitment relative to T907* | *PPARG-MED1 blockade assay EC₅₀ - %MED1 blockade relative to GW9662* | *HT1197 cell assay EC₅₀ - % inhibition of ANGPTL4* @ *100 nM* |
|---|---|---|---|
| **1** | B - A | B - A | ND - 91 |
| **2** | A - A | A - A | 0.14 - 91 |
| **3** | A - A | A - A | 0.09 - 95 |
| **4** | A - A | A - A | ND - 88 |
| **5** | B - B | B - A | ND - 71 |
| **6** | A - A | A - A | ND - 73 |
| **7** | E - A | E - A | ND - 23 |
| **8** | E - A | E - A | ND - ND |
| **9** | C - B | C - A | ND - 22 |
| **10** | C - A | C - A | ND - 88 |
| **11** | D - A | D - A | ND - 58 |
| **12** | E - B | E - B | ND - 18 |
| **13** | B - A | B - A | ND - 78 |
| **14** | B - A | B - A | ND - 88 |
| **15** | B - A | B - A | 3.1 - 81 |
| **16** | B - A | B - A | ND - 85 |
| **17** | B - A | B - A | ND - 78 |
| **18** | B - A | B - A | ND -57 |
| **19** | B - A | B - A | ND - 84 |
| **20** | B - A | B - A | ND - 80 |
| **21** | A - A | A - A | ND - 81 |
| **22** | A - A | B - A | ND - 91 |
| **23** | A - A | A - A | ND - 76 |
| **24** | B - A | B - A | ND - 87 |
| **25** | A - A | A - A | ND - 86 |
| **26** | A - A | A - A | ND - 85 |
| **27** | B - A | B - A | ND - 70 |
| **28** | B - A | B - A | ND - 79 |
| **29** | A - A | A - A | 0.49 - 90 |
| **30** | A - A | A - A | ND - 88 |
| **31** | A - A | A - A | ND - 86 |
| **32** | A - A | A - A | ND - 87 |
| **33** | A - A | A - A | ND - 31 |
| **34** | A - A | A - A | ND - 87 |

While we have described a number of embodiments, it is apparent that our basic examples may be altered to provide other embodiments that utilize the compounds and methods of this invention. Therefore, it will be appreciated that the scope of this invention is to be defined by the appended claims rather than by the specific embodiments that have been represented by way of example.

Unless otherwise defined, all technical and scientific terms used herein are accorded the meaning commonly known to one with ordinary skill in the art.

## Claims

1. A compound having the Formula I: 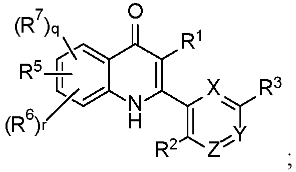 or a pharmaceutically acceptable salt thereof, wherein
X, Y, and Z are each independently N or -CR⁴, wherein at least one of X, Y, or Z is N;
R¹ is hydrogen, halo, (C₁-C₄)alkyl, or hydroxyl;
R² is halo, -SR^{g}, -SOR^{g}, -SO₂R^{g}, or -OR^{g};
R³ is cyano or nitro;
R⁴ is hydrogen, halo, (C₁-C₄)alkyl, (C₁-C₄)alkoxy, or hydroxyl;
R⁵ is halo, halo(C₁-C₄)alkyl, or cyano;
R⁶ is halo, halo(C₁-C₄)alkyl, (C₁-C₄)alkyl, or cyano;
R⁷ is halo, (C₁-C₄)alkyl, (C₁-C₄)alkoxy, halo(C₁-C₄)alkyl, halo(C₁-C₄)alkoxy, -(C₁-C₄)alkylOR^{a}, -(C₁-C₄)alkylC(O)R^{a}, -(C₁-C₄)alkylC(O)OR^{a}, -C(O)NR^{a}R^{b}, -(C₁-C₄)alkylC(O)NR^{a}R^{b}, -C(O)R^{a}, -C(O)OR^{a}, -NR^{a}R^{b}, -(C₁-C₄)alkylNR^{a}R^{b}, -C(O)NR^{a}SO₃H, - NR^{a}C(O)R^{b}, -NR^{a}C(O)OR^{b}, -NR^{a}C(S)OR^{b}, -NR^{c}C(O)N^{a}R^{b}, -NR^{c}C(S)NR^{a}R^{b}, -NR^{c}S(O)₂NR^{a}R^{b}, -C(S)R^{a}, -S(O)₂R^{a}, -S(O)R^{a}, -C(S)OR^{a}, -C(S)NR^{a}R^{b}, -NR^{a}C(S)R^{b}, -SR^{a}, phenyl, 4- to 6-membered heterocyclyl, and 5- to 7-membered heteroaryl, wherein each of said phenyl, 4- to 6-membered heterocyclyl, and 5- to 7-membered heteroaryl are optionally and independently substituted with 1 to 3 groups selected from R⁸;
R⁸ is selected from halo, (C₁-C₄)alkyl, halo(C₁-C₄)alkyl, (C₁-C₄)alkoxy, halo(C₁-C₄)alkoxy, nitro, oxo, cyano, -(C₁-C₄)alkylOR^{d}, -(C₁-Ca₄)alkylC(O)R^{d}, -(C₁-C₄)alkylC(O)OR^{d}, - C(O)NR^{d}R^{e}, -(C₁-C₄)alkylC(O)NR^{d}R^{e}, -C(O)R^{d}, -C(O)OR^{d}, -NR^{d}R^{e}, -(C₁-C₄)alkylNR^{d}R^{e}, - C(O)NR^{d}SO₃H, -NR^{d}C(O)R^{e}, -NR^{d}C(O)OR^{e}, -NR^{d}C(S)OR^{e}, -NR^{f}C(O)N^{d}R^{e}, -NR^{f}C(S)NR^{d}R^{e}, - NR^{f}S(O)₂NR^{d}R^{e}, -C(S)R^{d}, -S(O)₂R^{d}, -S(O)R^{d}, -C(S)OR^{d}, -C(S)NR^{d}R^{e}, -NR^{d}C(S)R^{e}, and -SR^{d};
R^{a}, R^{b}, R^{c}, R^{d}, R^{e}, R^{f}, and R^{g} are each independently hydrogen or (C₁-C₄)alkyl optionally substituted with 1 or 2 -NR'R" groups wherein R' and R'' are each independently selected from hydrogen, (C₁-C₄)alkyl, and halo(C₁-C₄)alkyl; and
q and r are each independently 0 or 1.

2. The compound of Claim 1, wherein the compound is of the Formula II or Formula III: 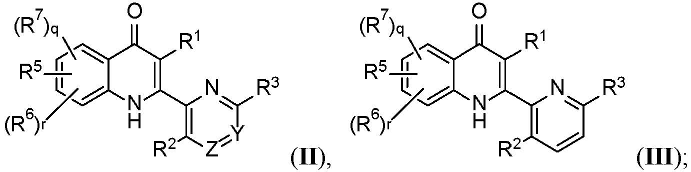 or a pharmaceutically acceptable salt thereof.

3. The compound of Claim 1 or 2, or a pharmaceutically acceptable salt thereof, wherein:
(i) R² is halo, -S(C₁-C₄)alkyl, -SO(C₁-C₄)alkyl, -SO₂(C₁-C₄)alkyl, or -O(C₁-C₄)alkylN[(C₁-C₄)alkyl]₂;
(ii) R² is chloro, -SCH₃, -SOCH₃, -SO₂CH₃, or -O(CH₂)₂N(CH₃)₂; or
(iii) R² is chloro.

4. The compound of any one of Claims 1 to 3, or a pharmaceutically acceptable salt thereof, wherein R³ is cyano.

5. The compound of any one of Claims 1 to 4, or a pharmaceutically acceptable salt thereof, wherein:
(i) R¹ is hydrogen, fluoro, hydroxyl, or methyl; or
(ii) R¹ is hydrogen.

6. The compound of any one of Claims 1 to 5, or a pharmaceutically acceptable salt thereof, wherein:
(i) R⁵ is halo or cyano; or
(ii) R⁵ is halo.

7. The compound of any one of Claims 1 to 6, or a pharmaceutically acceptable salt thereof, wherein q is 1.

8. The compound of any one of Claims 1 to 7, or a pharmaceutically acceptable salt thereof, wherein:
(i) r is 1; or
(ii) r is 0.

9. The compound of any one of Claims 1 to 8, or a pharmaceutically acceptable salt thereof, wherein R⁶ is halo.

10. The compound of any one of Claims 1 to 9, or a pharmaceutically acceptable salt thereof, wherein:
(i) R⁷ is halo, halo(C₁-C₄)alkyl, (C₁-C₄)alkyl, (C₁-C₄)alkoxy, -(C₁-C₄)alkylOR^{a}, - C(O)NR^{a}R^{b}, phenyl, 4- to 6-membered heterocyclyl, and 5- to 7-membered heteroaryl, wherein each of said phenyl, 4- to 6-membered heterocyclyl, and 5- to 7-membered heteroaryl are optionally and independently substituted with 1 to 3 groups selected from R⁸;
(ii) R⁷ is halo, halo(C₁-C₄)alkyl, (C₁-C₄)alkyl, (C₁-C₄)alkoxy, -(C₁-C₄)alkylOR^{a}, - C(O)NR^{a}R^{b}, phenyl, pyridinyl, piperazinyl, piperidinyl, pyrrolidinyl, thiomorpholinyl, pyrazolyl, and oxetanyl, wherein each of said phenyl, pyridinyl, pyrazolyl, pyrrolidinyl, piperazinyl, thiomorpholinyl, piperidinyl, and oxetanyl are optionally and independently substituted with 1 to 3 groups selected from R⁸; or
(iii) R⁷ is halo, halo(C₁-C₄)alkyl, (C₁-C₄)alkyl, (C₁-C₄)alkoxy, -(C₁-C₄)alkylOR^{a}, - C(O)NR^{a}R^{b}, phenyl, pyridinyl, pyrazolyl, and oxetanyl, wherein each of said phenyl, pyridinyl, pyrazolyl, and oxetanyl are optionally and independently substituted with 1 to 3 groups selected from R⁸.

11. The compound of any one of Claims 1 to 10, or a pharmaceutically acceptable salt thereof, wherein:
(i) R⁸ is selected from halo, -C(O)NR^{d}R^{e}, (C₁-C₄)alkyl, halo(C₁-C₄)alkyl, (C₁-C₄)alkoxy, halo(C₁-C₄)alkoxy, oxo, and cyano;
(ii) R⁸ is selected from halo, (C₁-C₄)alkyl, halo(C₁-C₄)alkyl, (C₁-C₄)alkoxy, halo(C₁-C₄)alkoxy, oxo, and cyano;
(iii) R⁸ is selected from -C(O)NR^{d}R^{e}, (C₁-C₄)alkyl, and oxo;
(iv) R⁸ is selected from -C(O)N(CH₃)₂, CH₃, and oxo; or
(v) R⁸ is halo(C₁-C₄)alkyl.

12. The compound of Claim 1, wherein the compound is of the structural formula: 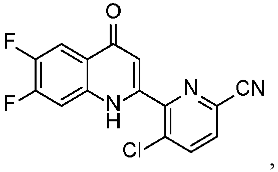 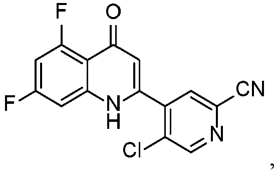 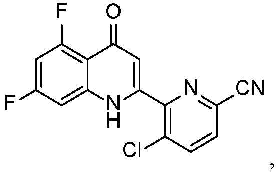 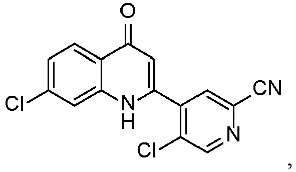 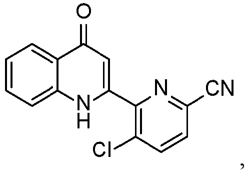 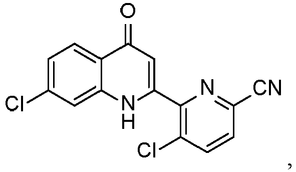 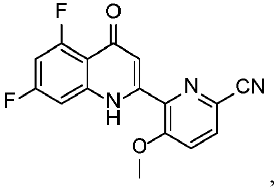 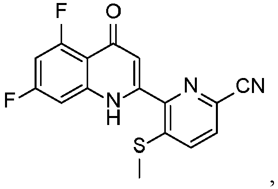 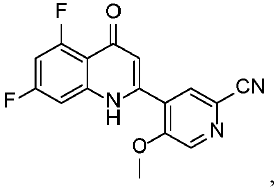 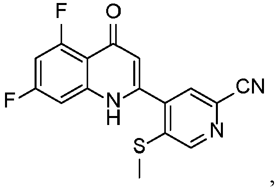 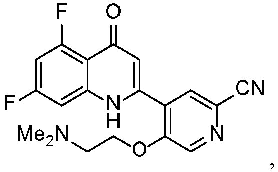 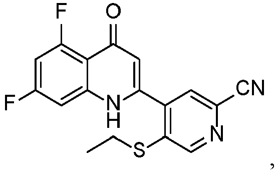 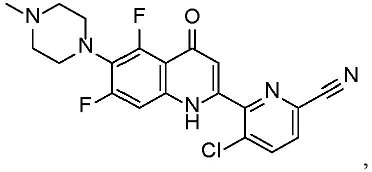 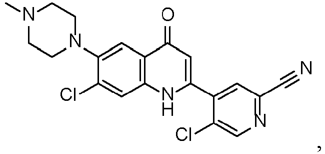 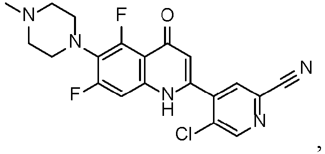 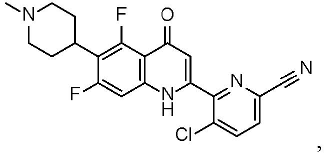 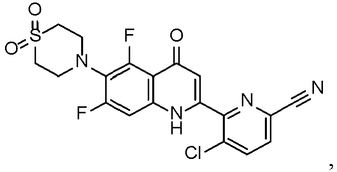 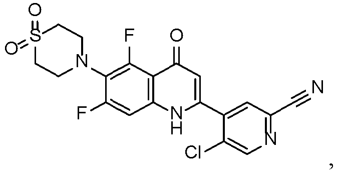 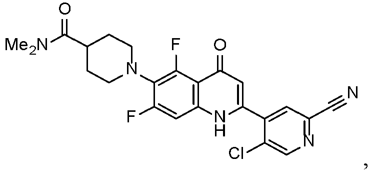 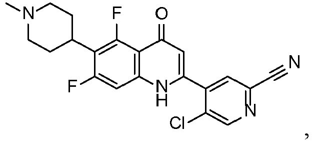 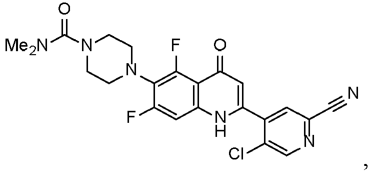 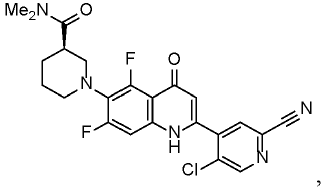 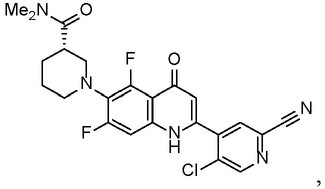 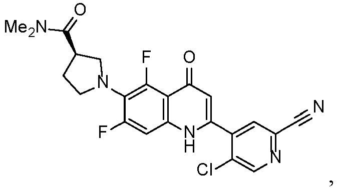 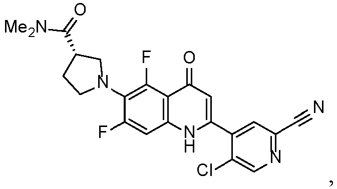 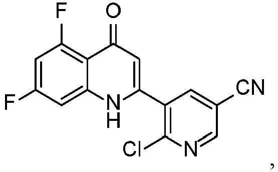 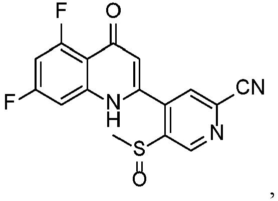 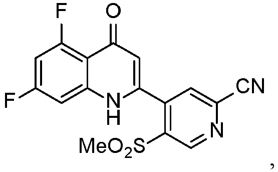 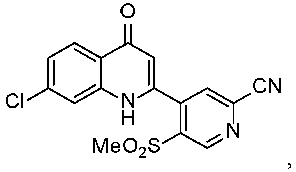 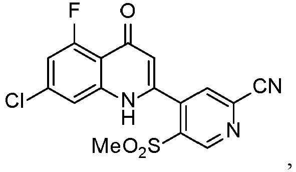 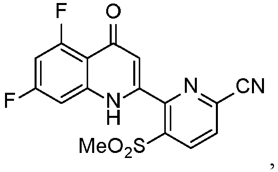 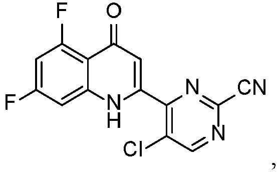 or 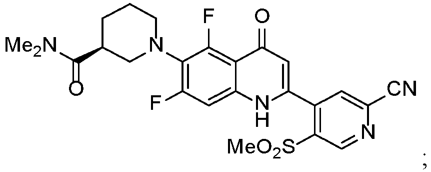 or a pharmaceutically acceptable salt of any of the foregoing.

13. A pharmaceutical composition comprising a compound of any one of Claims 1 to 12, or a pharmaceutically acceptable salt thereof, and a pharmaceutically acceptable carrier.

14. A compound of any one of Claims 1 to 12, or a pharmaceutically acceptable salt thereof, or the composition of Claim 13, for use in treating a cancer responsive to the suppression of **PPARG** in a subject.

15. The compound for use of Claim 14, wherein:
(i) the cancer is selected from breast cancer, pancreatic cancer, ovarian cancer, prostate cancer, renal cancer, bladder cancer, testicular cancer, urothelial cancer, skin cancer, melanoma, colon cancer, kidney cancer, brain cancer and a hematopoietic cancer; or
(ii) the cancer is bladder cancer.

## Patentansprüche

1. Verbindung mit der Formel I: 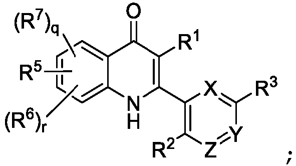 oder ein pharmazeutisch annehmbares Salz davon, wobei
X, Y und Z jeweils unabhängig voneinander N oder - CR⁴ sind, wobei mindestens eines von X, Y oder Z N ist;
R¹ Wasserstoff, Halogen, (C₁-C₄)-Alkyl oder Hydroxyl ist;
R² Halogen, -SR^{g}, -SOR^{g}, -SO₂R^{g} oder -OR^{g} ist;
R³ Cyano oder Nitro ist;
R⁴ Wasserstoff, Halogen, (C₁-C₄)-Alkyl, (C₁-C₄)-Alkoxy oder Hydroxyl ist;
R⁵ Halogen, Halogen- (C₁-C₄)-alkyl oder Cyano ist;
R⁶ Halogen, Halogen- (C₁-C₄)-alkyl, (C₁-C₄)-Alkyl oder Cyano ist;
R⁷ Halogen, (C₁-C₄)-Alkyl, (C₁-C₄)-Alkoxy, Halogen-(C₁-C₄)-alkyl, Halogen-(C₁-C₄)-alkoxy, -(C₁-C₄)-alkyl-OR^{a}, -(C₁-C₄)-alkyl-C(O)R^{a}, -(C₁-C₄)-alkyl-C(O)OR^{a}, -C(O)NR^{a}R^{b}, -(C₁-C₄)-alkyl-C(O)NR^{a}R^{b}, -C(O)R^{a}, -C(O)OR^{a}, -NR^{a}R^{b}, -(C₁-C₄)-alkyl-NR^{a}R^{b}, -C(O)NR^{a}SO₃H, -NR^{a}C(O)R^{b}, -NR^{a}C(O)OR^{b}, - NR^{a}C(S)OR^{b}, -NR^{c}C(O)N^{a}R^{b}, -NR^{c}C(S)NR^{a}R^{b}, -NR^{c}S(O)₂NR^{a}R^{b}, - C(S)R^{a}, -S(O)₂R^{a}, -S(O)R^{a}, -C(S)OR^{a}, -C(S)NR^{a}R^{b}, - NR^{a}C(S)R^{b}, -SR^{a}, Phenyl, 4- bis 6-gliedriges Heterocyclyl und 5- bis 7-gliedriges Heteroaryl ist, wobei jedes von dem Phenyl, 4- bis 6-gliedrigen Heterocyclyl und 5- bis 7-gliedrigen Heteroaryl optional und unabhängig voneinander mit 1 bis 3 Gruppen, ausgewählt aus R⁸, substituiert ist;
R⁸ ausgewählt ist aus Halogen, (C₁-C₄)-Alkyl, Halogen-(C₁-C₄)-alkyl, (C₁-C₄)-Alkoxy, Halogen-(C₁-C₄)-alkoxy, Nitro, Oxo, Cyano, -(C₁-C₄)-alkyl-OR^{d}, -(C₁-C₄)-alkyl-C(O)R^{d}, -(C₁-C₄)-alkyl-C(O)OR^{d}, -C(O)NR^{d}R^{e}, -(C₁-C₄) - alkyl-C(O)NR^{d}R^{e}, -C(O)R^{d}, -C(O)OR^{d}, -NR^{d}R^{e}, -(C₁-C₄)-alkyl-NR^{d}R^{e}, -C(O)NR^{d}SO₃H, -NR^{d}C(O)R^{e}, -NR^{d}C(O)OR^{e}, -NR^{d}C(S)OR^{e}, -NR^{f}C(O)N^{d}R^{e}, -NR^{f}C(S)NR^{d}R^{e}, -NR^{f}S(O)₂NR^{d}R^{e}, -C(S)R^{d}, - S(O)₂R^{d}, -S(O)R^{d}, -C(S)OR^{d}, -C(S)NR^{d}R^{e}, -NR^{d}C(S)R^{e} und - SR^{d};
R^{a}, R^{b}, R^{c}, R^{d}, R^{e}, R^{f} und R^{g} jeweils unabhängig voneinander Wasserstoff oder (C₁-C₄)-Alkyl, optional substituiert mit 1 oder 2 -NR'R"-Gruppen, sind, wobei R' und R" jeweils unabhängig voneinander aus Wasserstoff, (C₁-C₄)-Alkyl und Halogen- (C₁-C₄)-alkyl ausgewählt sind; und
q und r jeweils unabhängig voneinander 0 oder 1 sind.

2. Verbindung nach Anspruch 1, wobei die Verbindung die Formel II oder Formel III besitzt: 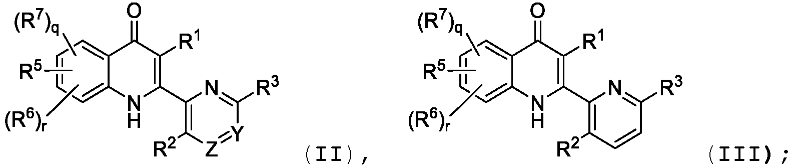 oder ein pharmazeutisch annehmbares Salz davon.

3. Verbindung nach Anspruch 1 oder 2 oder ein pharmazeutisch annehmbares Salz davon, wobei:
(i) R² Halogen, -S(C₁-C₄)-alkyl, -SO(C₁-C₄)-alkyl, - SO₂(C₁-C₄)-alkyl oder -O(C₁-C₄)-alkyl-N[(C₁-C₄)-alkyl] ₂ ist;
(ii) R² Chlor, -SCH₃, -SOCH₃, -SO₂CH₃ oder - O(CH₂)₂N(CH₃)₂ ist; oder
(iii) R² Chlor ist.

4. Verbindung nach irgendeinem der Ansprüche 1 bis 3 oder ein pharmazeutisch annehmbares Salz davon, wobei R³ Cyano ist.

5. Verbindung nach irgendeinem der Ansprüche 1 bis 4 oder ein pharmazeutisch annehmbares Salz davon, wobei:
(i) R¹ Wasserstoff, Fluor, Hydroxyl oder Methyl ist; oder
(ii) R¹ Wasserstoff ist.

6. Verbindung nach irgendeinem der Ansprüche 1 bis 5 oder ein pharmazeutisch annehmbares Salz davon, wobei:
(i) R⁵ Halogen oder Cyano ist; oder
(ii) R⁵ Halogen ist.

7. Verbindung nach irgendeinem der Ansprüche 1 bis 6 oder ein pharmazeutisch annehmbares Salz davon, wobei q 1 ist.

8. Verbindung nach irgendeinem der Ansprüche 1 bis 7 oder ein pharmazeutisch annehmbares Salz davon, wobei:
(i) r 1 ist; oder
(ii) r 0 ist.

9. Verbindung nach irgendeinem der Ansprüche 1 bis 8 oder ein pharmazeutisch annehmbares Salz davon, wobei R⁶ Halogen ist.

10. Verbindung nach irgendeinem der Ansprüche 1 bis 9 oder ein pharmazeutisch annehmbares Salz davon, wobei:
(i) R⁷ Halogen, Halogen- (C₁-C₄)-alkyl, (C₁-C₄)-Alkyl, (C₁-C₄)-Alkoxy, -(C₁-C₄)-alkyl-OR^{a}, -C(O)NR^{a}R^{b}, Phenyl, 4-bis 6-gliedriges Heterocyclyl und 5- bis 7-gliedriges Heteroaryl ist, wobei jedes von dem Phenyl, 4- bis 6-gliedrigen Heterocyclyl und 5- bis 7-gliedrigen Heteroaryl optional und unabhängig voneinander mit 1 bis 3 Gruppen, ausgewählt aus R⁸, substituiert ist;
(ii) R⁷ Halogen, Halogen- (C₁-C₄)-alkyl, (C₁-C₄)-Alkyl, (C₁-C₄)-Alkoxy, -(C₁-C₄)-alkyl-OR^{a}, -C(O)NR^{a}R^{b}, Phenyl, Pyridinyl, Piperazinyl, Piperidinyl, Pyrrolidinyl, Thiomorpholinyl, Pyrazolyl und Oxetanyl ist, wobei jedes von dem Phenyl, Pyridinyl, Pyrazolyl, Pyrrolidinyl, Piperazinyl, Thiomorpholinyl, Piperidinyl und Oxetanyl optional und unabhängig voneinander mit 1 bis 3 Gruppen, ausgewählt aus R⁸, substituiert ist; oder
(iii) R⁷ Halogen, Halogen- (C₁-C₄)-alkyl, (C₁-C₄)-Alkyl, (C₁-C₄)-Alkoxy, - (C₁-C₄)-alkyl-OR^{a}, -C(O)NR^{a}R^{b}, Phenyl, Pyridinyl, Pyrazolyl und Oxetanyl ist, wobei jedes von dem Phenyl, Pyridinyl, Pyrazolyl und Oxetanyl optional und unabhängig voneinander mit 1 bis 3 Gruppen, ausgewählt aus R⁸, substituiert ist.

11. Verbindung nach irgendeinem der Ansprüche 1 bis 10 oder ein pharmazeutisch annehmbares Salz davon, wobei:
(i) R⁸ ausgewählt ist aus Halogen, -C(O)NR^{d}R^{e}, (C₁-C₄)-Alkyl, Halogen- (C₁-C₄)-alkyl, (C₁-C₄)-Alkoxy, Halogen-(C₁-C₄)-alkoxy, Oxo und Cyano;
(ii) R⁸ ausgewählt ist aus Halogen, (C₁-C₄)-Alkyl, Halogen- (C₁-C₄)-alkyl, (C₁-C₄)-Alkoxy, Halogen- (C₁-C₄)-alkoxy, Oxo und Cyano;
(iii) R⁸ ausgewählt ist aus -C(O)NR^{d}R^{e}, (C₁-C₄)-Alkyl und Oxo;
(iv) R⁸ ausgewählt ist aus -C(O)N(CH₃)₂, CH₃ und Oxo; oder
(v) R⁸ Halogen- (C₁-C₄)-alkyl ist.

12. Verbindung nach Anspruch 1, wobei die Verbindung folgende Strukturformel besitzt: 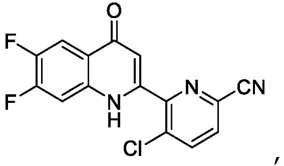 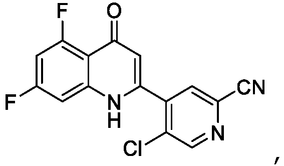 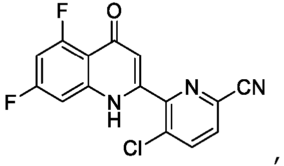 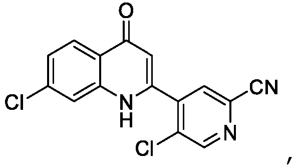 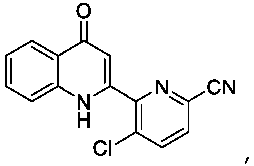 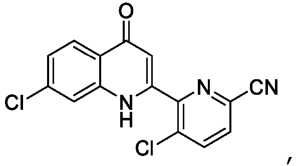 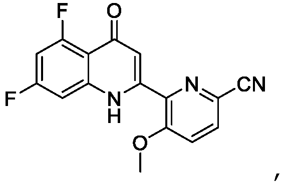 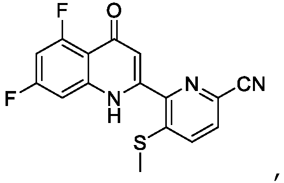 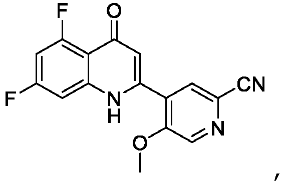 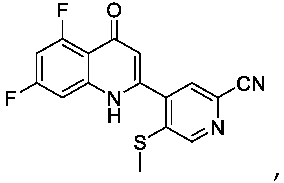 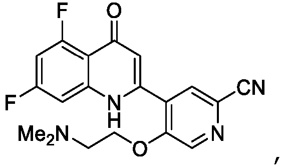 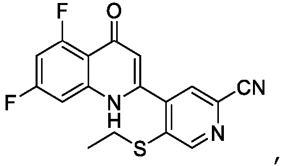 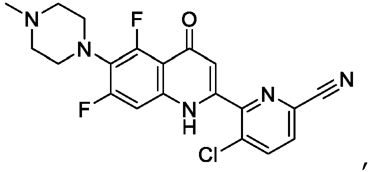 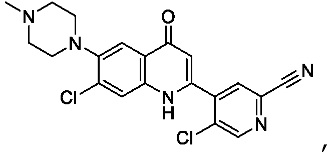 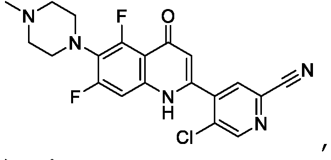 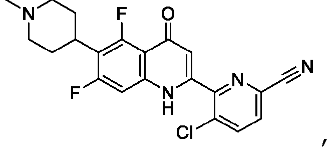 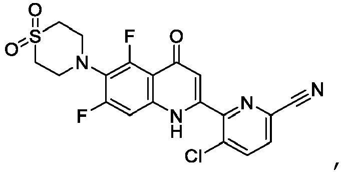 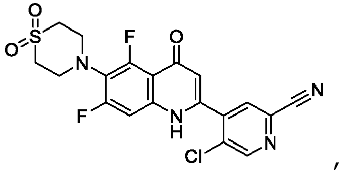 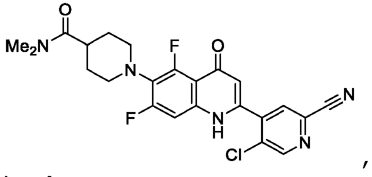 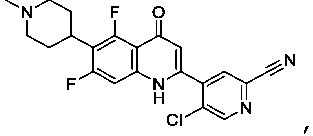 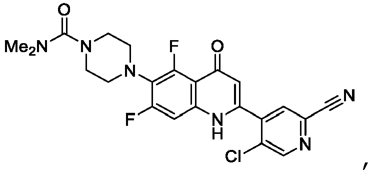 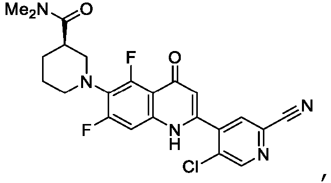 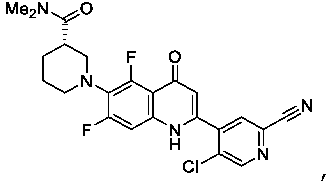 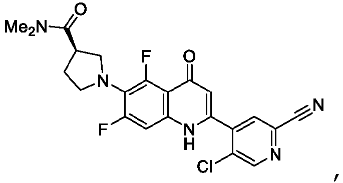 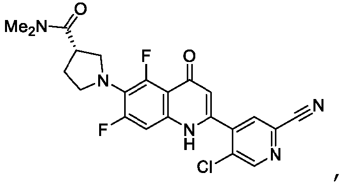 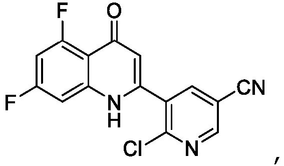 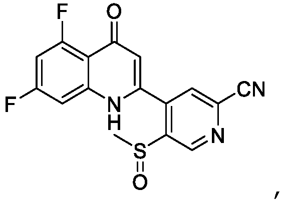 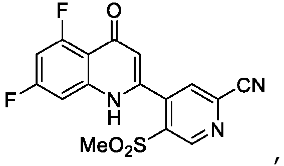 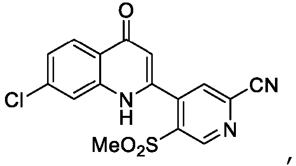 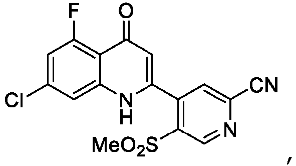 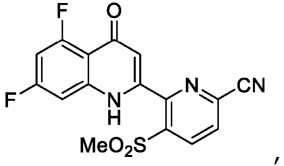 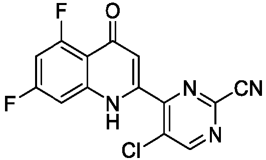 oder 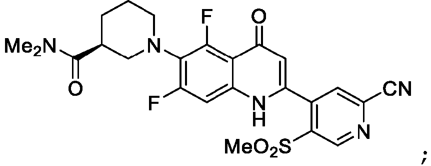 oder ein pharmazeutisch annehmbares Salz von irgendeinem des Vorstehenden.

13. Pharmazeutische Zusammensetzung, umfassend eine Verbindung nach irgendeinem der Ansprüche 1 bis 12 oder ein pharmazeutisch annehmbares Salz davon; und einen pharmazeutisch annehmbaren Träger.

14. Verbindung nach irgendeinem der Ansprüche 1 bis 12 oder ein pharmazeutisch annehmbares Salz davon oder die Zusammensetzung nach Anspruch 13 zur Verwendung bei der Behandlung einer Krebserkrankung, die auf die Unterdrückung von PPARG bei einem Subjekt anspricht.

15. Verbindung zur Verwendung nach Anspruch 14, wobei:
(i) die Krebserkrankung ausgewählt ist aus Brustkrebs, Bauchspeicheldrüsenkrebs, Eierstockkrebs, Prostatakrebs, Nierenkrebs, Blasenkrebs, Hodenkrebs, Urothelkrebs, Hautkrebs, Melanom, Dickdarmkrebs, Nierenkrebs, Hirnkrebs und einem hämatopoetischen Krebs; oder
(ii) die Krebserkrankung Blasenkrebs ist.

## Revendications

1. Composé ayant la formule I : 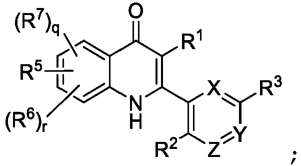 ou sel pharmaceutiquement acceptable de celui-ci, dans lequel
X, Y et Z sont chacun indépendamment N ou -CR⁴, dans lequel au moins l'un parmi X, Y ou Z est N ;
R¹ représente l'hydrogène, le groupe halo, alkyle (C₁-C₄)ou hydroxyle ;
R² représente le groupe halo, -SR^{g}, -SOR^{g}, -SO₂R^{g} ou -OR^{g} ;
R³ représente le groupe cyano ou nitro ;
R⁴ représente l'hydrogène, le groupe halo, alkyle (C₁-C₄), alcoxyle (C₁-C₄) ou hydroxyle ;
R⁵ représente le groupe halo, haloalkyle (C₁-C₄) ou cyano ;
R⁶ représente le groupe halo, haloalkyle(C₁-C₄), alkyle(C₁-C₄) ou cyano ;
R⁷ représente les groupes halo, alkyle (C₁-C₄), alcoxyle(C₁-C₄), haloalkyle (C₁-C₄), haloalcoxyle(C₁-C₄), - (C₁-C₄)alkylOR^{a}, - (C₁-C₄)alkylC(O)R^{a}, - (C₁-C₄) alkylC(O)OR^{a}, -C(O)NR^{a}R^{b}, -(C₁-C₄)alkylC(O)NR^{a}R^{b}, -C(O)R^{a}, -C(O)OR^{a}, - NR^{a}R^{b}, - (C₁-C₄)alkylNR^{a}R^{b}, -C(O)NR^{a}SO₃H, -NR^{a}C(O)R^{b}, - NR^{a}C(O)OR^{b}, -NR^{a}C(S)OR^{b}, -NR^{c}C(O)N^{a}R^{b}, -NR^{c}C(S)NR^{a}R^{b}, - NR^{c}S(O)₂NR^{a}R^{b}, -C(S)R^{a}, -S(O)₂R^{a}, -S(O)R^{a}, -C(S)OR^{a}, - C(S)NR^{a}R^{b}, -NR^{a}C(S)R^{b}, -SR^{a}, phényle, hétérocyclyle de 4 à 6 chaînons et hétéroaryle de 5 à 7 chaînons, dans lequel chacun parmi lesdits phényle, hétérocyclyle de 4 à 6 chaînons et hétéroaryle de 5 à 7 chaînons est éventuellement et indépendamment substitué par 1 à 3 groupes sélectionnés dans R⁸;
R⁸ est sélectionné parmi les groupes halo, alkyle (C₁-C₄), haloalkyle (C₁-C₄), alcoxyle (C₁-C₄), haloalcoxyle (C₁-C₄), nitro, oxo, cyano, - (C₁-C₄)alkylOR^{d}, -(C₁-C₄)alkylC(O)R^{d}, -(C₁-C₄)alkylC(O)OR^{d}, -C(O)NR^{d}R^{e}, -(C₁-C₄)alkylC(O)NR^{d}R^{e}, -C(O)R^{d}, -C(O)OR^{d}, -NR^{d}R^{e}, -(C₁-C₄)alkylNR^{d}R^{e}, -C(O)NR^{d}SO₃H, -NR^{d}C(O)R^{e}, -NR^{d}C(O)OR^{e}, - NR^{d}C(S)OR^{e}, -NR^{f}C(O)N^{d}R^{e}, -NR^{f}C(S)NR^{d}R^{e}, -NR^{f}S(O)₂NR^{d}R^{e}, - C(S)R^{d}, -S(O)₂R^{d}, -S(O)R^{d}, -C(S)OR^{d}, -C(S)NR^{d}R^{e}, -NR^{d}C(S)R^{e} et -SR^{d} ;
R^{a}, R^{b}, R^{c}, R^{d}, R^{e}, R^{f} et R^{g} sont chacun indépendamment l'hydrogène ou le groupe alkyle (C₁-C₄) éventuellement substitué par 1 ou 2 groupes -NR'R'' dans lesquels R' et R" sont chacun indépendamment sélectionnés parmi l'hydrogène, l'alkyle (C₁-C₄), et l'haloalkyle (C₁-C₄) ; et
q et r sont chacun indépendamment 0 ou 1.

2. Composé selon la revendication 1, dans lequel le composé est représenté par la Formule II ou la Formule III : 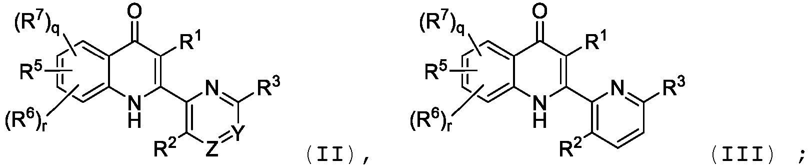 ou sel pharmaceutiquement acceptable de celui-ci.

3. Composé selon la revendication 1 ou 2, ou sel pharmaceutiquement acceptable de celui-ci, dans lequel :
(i) R² représente le groupe halo, -S(C₁-C₄)alkyl, - SO(C₁-C₄)alkyle, -SO₂(C₁-C₄)alkyle ou -O(C₁-C₄)alkyleN[(C₁-C₄)alkyle]₂ ;
(ii) R² représente le groupe chloro, -SCH₃, -SOCH₃, -SO₂CH₃ ou -O(CH₂)₂N(CH₃)₂ ; ou
(iii) R² représente le groupe chloro.

4. Composé selon l'une quelconque des revendications 1 à 3, ou sel pharmaceutiquement acceptable de celui-ci, dans lequel R³ représente le groupe cyano.

5. Composé selon l'une quelconque des revendications 1 à 4, ou sel pharmaceutiquement acceptable de celui-ci, dans lequel :
(i) R¹ représente l'hydrogène, le groupe fluoro, hydroxyle ou méthyle ; ou
(ii) R¹ représente l'hydrogène.

6. Composé selon l'une quelconque des revendications 1 à 5, ou sel pharmaceutiquement acceptable de celui-ci, dans lequel :
(i) R⁵ représente le groupe halo ou cyano ; ou
(ii) R⁵ représente le groupe halo.

7. Composé selon l'une quelconque des revendications 1 à 6, ou sel pharmaceutiquement acceptable de celui-ci, dans lequel q est 1.

8. Composé selon l'une quelconque des revendications 1 à 7, ou sel pharmaceutiquement acceptable de celui-ci, dans lequel :
(i) r est 1 ; ou
(ii) r est 0.

9. Composé selon l'une quelconque des revendications 1 à 8, ou sel pharmaceutiquement acceptable de celui-ci, dans lequel R⁶ représente le groupe halo.

10. Composé selon l'une quelconque des revendications 1 à 9, ou sel pharmaceutiquement acceptable de celui-ci, dans lequel :
(i) R⁷ représente les groupes halo, haloalkyle(C₁-C₄), alkyle(C₁-C₄), alcoxyle(C₁-C₄), -(C₁-C₄)alkylOR^{a}, - C(O)NR^{a}R^{b}, phényle, hétérocyclyle de 4 à 6 chaînons et hétéroaryle de 5 à 7 chaînons, dans lequel chacun parmi lesdits phényle, hétérocyclyle de 4 à 6 chaînons et hétéroaryle de 5 à 7 chaînons est éventuellement et indépendamment substitué par 1 à 3 groupes sélectionnés dans R⁸ ;
(ii) R⁷ représente les groupes halo, haloalkyle(C₁-C₄), alkyle (C₁-C₄), alcoxyle(C₁-C₄), - (C₁-C₄)alkylOR^{a}, - C(O)NR^{a}R^{b}, phényle, pyridinyle, pipérazinyle, pipéridinyle, pyrrolidinyle, thiomorpholinyle, pyrazolyle et oxétanyle, dans lequel chacun parmi lesdits phényle, pyridinyle, pyrazolyle, pyrrolidinyle, pipérazinyle, thiomorpholinyle, pipéridinyle et oxétanyle est éventuellement et indépendamment substitué par 1 à 3 groupes sélectionnés dans R⁸ ; ou
(iii) R⁷ représente les groupes halo, haloalkyle(C₁-C₄), alkyle (C₁-C₄), alcoxyle(C₁-C₄), - (C₁-C₄)alkylOR^{a}, - C(O)NR^{a}R^{b}, phényle, pyridinyle, pyrazolyle et oxétanyle, dans lequel chacun parmi lesdits phényle, pyridinyle, pyrazolyle et oxétanyle est éventuellement et indépendamment substitué par 1 à 3 groupes sélectionnés dans R⁸ ;

11. Composé selon l'une quelconque des revendications 1 à 10, ou sel pharmaceutiquement acceptable de celui-ci, dans lequel :
(i) R⁸ est sélectionné parmi les groupes halo, - C(O)NR^{d}R^{e}, alkyle (C₁-C₄), haloalkyle (C₁-C₄), alcoxyle(C-C₄), haloalcoxyle (C₁-C₄), oxo et cyano ;
(ii) R⁸ est sélectionné parmi les groupes halo, alkyle (C₁-C₄), haloalkyle (C₁-C₄), alcoxyle(C₁-C₄), haloalcoxyle(C₁-C₄), oxo et cyano ;
(iii) R⁸ est sélectionné parmi les groupes - C(O)NR^{d}R^{e}, alkyle(C₁-C₄) et oxo ;
(iv) R⁸ est sélectionné parmi les groupes - C(O)N(CH₃)₂, CH₃ et oxo ; ou
(v) R⁸ représente le groupe alkyle (C₁-C₄).

12. Composé selon la revendication 1, dans lequel le composé est représenté par la formule structurelle : 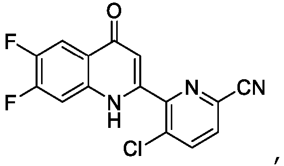 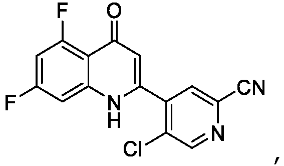 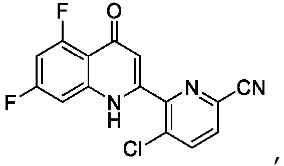 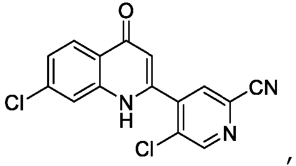 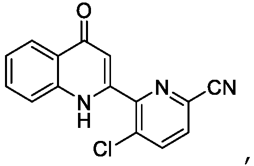 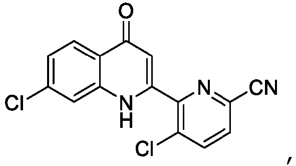 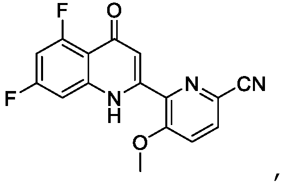 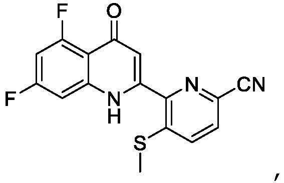 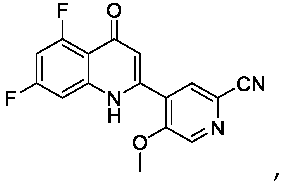 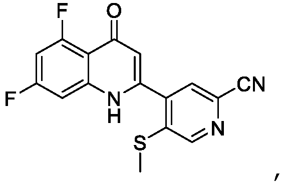 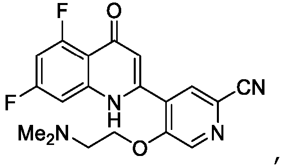 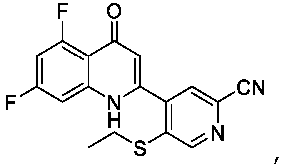 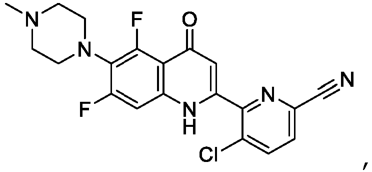 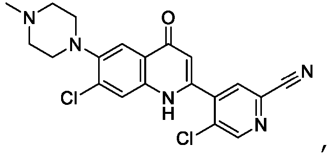 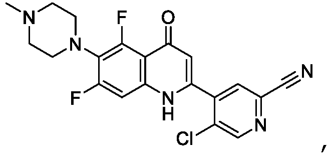 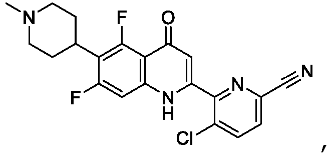 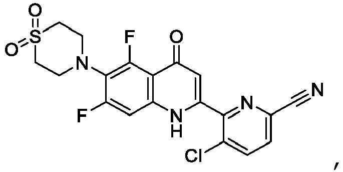 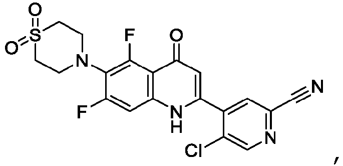 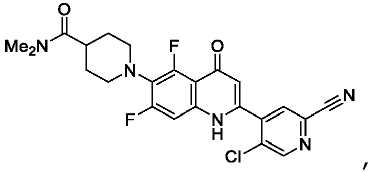 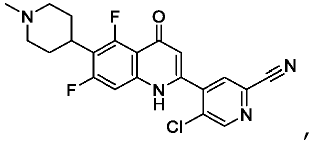 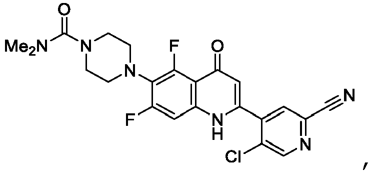 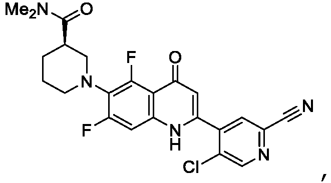 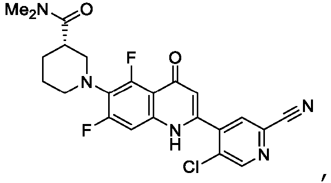 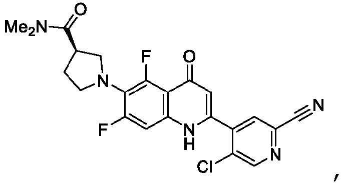 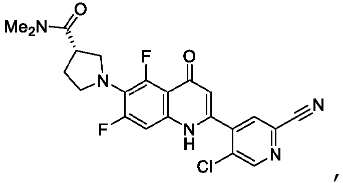 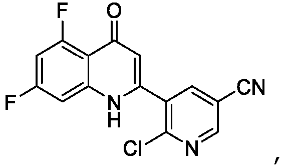 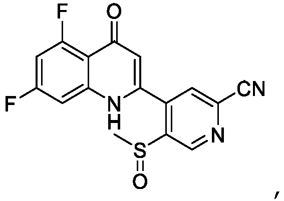 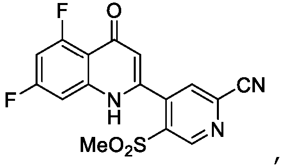 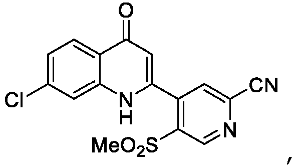 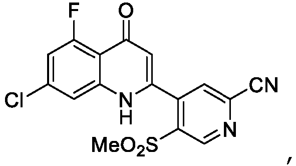 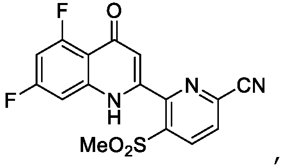 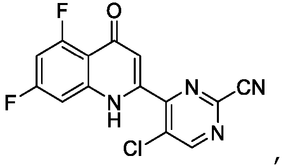 ou 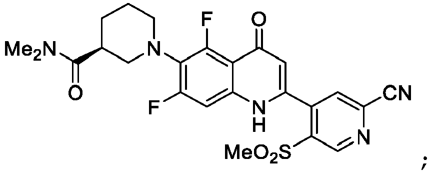 ou sel pharmaceutiquement acceptable selon l'un quelconque des éléments précédents.

13. Composition pharmaceutique comprenant un composé selon l'une quelconque des revendications 1 à 12, ou sel pharmaceutiquement acceptable de celle-ci ; et support pharmaceutiquement acceptable.

14. Composé selon l'une quelconque des revendications 1 à 12, ou sel pharmaceutiquement acceptable de celui-ci, ou composition selon la revendication 13, destiné à être utilisé dans le traitement d'un cancer en réponse à la suppression du PPARG chez un sujet.

15. Composé destiné à être utilisé selon la revendication 14, dans lequel :
(i) le cancer est sélectionné parmi le cancer du sein, le cancer du pancréas, le cancer de l'ovaire, le cancer de la prostate, le cancer du rein, le cancer de la vessie, le cancer du testicule, le cancer urothélial, le cancer de la peau, le mélanome, le cancer du côlon, le cancer du rein, le cancer du cerveau et un cancer hématopoïétique ; ou
(ii) le cancer est le cancer de la vessie.
